(19) **Europäisches Patentamt / European Patent Office / Office européen des brevets**

(11) **EP 2 984 209 B1**

(12) # FASCICULE DE BREVET EUROPEEN

(45) Date de publication et mention de la délivrance du brevet:
**19.06.2019 Bulletin 2019/25**

(21) Numéro de dépôt: **14721521.4**

(22) Date de dépôt: **07.04.2014**

(51) Int Cl.:
**C25B 9/18** (2006.01)   **C25B 1/12** (2006.01)

(86) Numéro de dépôt international:
**PCT/IB2014/060481**

(87) Numéro de publication internationale:
**WO 2014/167477 (16.10.2014 Gazette 2014/42)**

(54) **PROCEDES D'OBTENTION DE GAZ COMBUSTIBLE A PARTIR D'ELECTROLYSE DE L'EAU (EHT) OU DE CO-ELECTROLYSE AVEC H2O/CO2 AU SEIN D'UNE MEME ENCEINTE, REACTEUR CATALYTIQUE ET SYSTEME ASSOCIES**

VERFAHREN ZUR HERSTELLUNG VON BRENNGAS AUS WASSERELEKTROLYSE ODER CO-ELEKTROLYSE MIT H2O/CO2 IN DERSELBEN KAMMER, ZUGEHÖRIGER KATALYTISCHER REAKTOR UND SYSTEM

METHODS FOR PRODUCING COMBUSTIBLE GAS FROM THE ELECTROLYSIS OF WATER (HTE) OR CO-ELECTROLYSIS WITH H2O/CO2 IN THE SAME CHAMBER, AND ASSOCIATED CATALYTIC REACTOR AND SYSTEM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **08.04.2013 FR 1353104**

(43) Date de publication de la demande:
**17.02.2016 Bulletin 2016/07**

(73) Titulaire: **Commissariat à l'Énergie Atomique et aux Énergies Alternatives 75015 Paris (FR)**

(72) Inventeurs:
- **REYTIER, Magali**
  **F-38250 Villard De Lans (FR)**
- **ROUX, Guilhem**
  **F-38120 Saint-egreve (FR)**

(74) Mandataire: **Nony**
**11 rue Saint-Georges**
**75009 Paris (FR)**

(56) Documents cités:
**US-A1- 2007 163 889      US-A1- 2012 077 099**
**US-A1- 2012 171 596      US-A1- 2012 325 654**
**US-B1- 6 183 703**

EP 2 984 209 B1

**Description**

Domaine technique

La présente invention concerne le domaine d'obtention d'un gaz combustible choisi parmi le méthane, méthanol, le diméthyléther (DME) ou le diesel par catalyse hétérogène.

Les procédés d'obtention selon l'invention comportent une étape d'électrolyse de l'eau à haute température (EHT, ou EVHT pour électrolyse de la vapeur d'eau à haute température, ou HTE acronyme anglais pour « *High Temperature Electrolysis* », ou encore HTSE acronyme anglais pour « *High Temperature Steam Electrolysis* ») ou une étape dite de co-électrolyse de l'eau et dioxyde de carbone $CO_2$ à haute température et une étape de fabrication de gaz combustible par réaction catalytique.

L'invention a trait plus particulièrement à une nouvelle conception de réacteur dont l'enceinte sous pression loge à la fois un réacteur d'électrolyse, ou électrolyseur, à haute température (EHT), à empilement de cellules d'électrolyse élémentaires pour produire soit de l'hydrogène, soit un gaz de synthèse (*"syngas"* en anglais pour un mélange H2 + CO) à partir de vapeur d'eau H2O et de dioxyde de carbone CO2 et au moins un catalyseur agencé en aval de la sortie de l'électrolyseur pour convertir par catalyse hétérogène en gaz combustible souhaité, le gaz de synthèse obtenu préalablement soit directement du réacteur d'électrolyse soit indirectement par mélange de l'hydrogène produit avec du dioxyde de carbone $CO_2$ injecté dans l'enceinte.

Art antérieur

Parmi les solutions de stockage massif d'énergie déjà envisagées, le stockage hydraulique est déjà largement répandu. Les capacités restantes de ce type de stockage risquent d'être rapidement saturées. En outre, les systèmes hydrauliques nécessitent des conditions géographiques et géologiques particulières et peuvent de ce fait s'avérer assez onéreuses. Pour les problèmes de stockage à venir, le stockage hydraulique ne pourra donc être qu'une solution partielle.

Une solution alternative de stockage a également été envisagée : il s'agit du stockage par air comprimé (CAES acronyme anglais pour « *Compressed Air Energy Storage* »). Selon cette technologie, il est prévu de stocker de l'air comprimé produit avec l'électricité dans des cavités sous-terraines. Ces dernières exigent elles aussi des caractéristiques géographiques spécifiques, comme les cavités salines. Toutefois, le rendement de cette solution de stockage n'est pas satisfaisant.

L'hydrogène enfin est annoncé comme un vecteur d'énergie susceptible de pouvoir stocker massivement l'électricité dans certaines configurations : on peut citer ici le projet déjà réalisé en corse sous l'acronyme MYRTE (acronyme de Mission hYdrogène Renouvelable pour l'Intégration au Réseau Electrique) à l'initiative de la demanderesse.

Cependant, toutes ces solutions de stockage massif d'énergie requièrent des infrastructures importantes à développer (sites propres pour l'hydraulique, cavités sous-terraines, systèmes de stockage hydrogène). C'est pourquoi, plus récemment, le stockage massif d'énergie par conversion de l'électricité renouvelable en énergie chimique via la production de combustible de synthèse a fait une percée significative, représentant une alternative de stockage à fort potentiel. On peut citer ici la demande de brevet US 2009/0289227 qui évoque des solutions techniques de conversion.

Par ailleurs, réduire les émissions de dioxyde de carbone CO2 résultant de l'utilisation d'énergies fossiles, valoriser autant que possible le CO2 issu de l'utilisation de ces énergies plutôt que le stocker pour une durée indéterminée, utiliser à la demande l'électricité issue de sources d'énergie dite décarbonée, notamment lors des périodes de surproduction, transformer cette électricité en un produit stockable permettant éventuellement de produire de l'électricité à la demande lors des périodes de déficits de production sans avoir à faire appel à des énergies fortement carbonées sont autant d'objectifs à atteindre dans un souci d'efficacité globale.

La fabrication d'un gaz combustible de synthèse à partir d'un mélange de vapeur d'eau et de dioxyde de carbone CO2, grâce à de l'électricité dite décarbonée, répond à ces objectifs.

L'électrolyse de la vapeur d'eau H2O pour produire de l'hydrogène H2 et/ou la co-électrolyse de H2O+CO2 à haute température au sein d'un électrolyseur à oxydes solides est une des possibilités. Les réactions d'électrolyse de la vapeur d'eau (I) et de co-électrolyse de H2O+CO2 (II) se font selon les équations suivantes :

Electrolyse de l'eau: H2O →H2 + 1/2O2       (I)

Co-électrolyse: CO2 + H2O →CO + H2 + O2       (II).

Ainsi, l'électrolyse de la vapeur d'eau H2O →permet une fabrication dite directe du gaz combustible par catalyse hétérogène par injection d'un mélange d'hydrogène H2 produit selon l'électrolyse (I) et de dioxyde de carbone CO2 dans un catalyseur.

La co-électrolyse de H2O+CO2 permet une fabrication dite indirecte du gaz combustible à partir du gaz de synthèse ($H_2$ + CO) produit selon la co-électrolyse (II).

Le gaz combustible ainsi fabriqué peut être un hydrocarbure et notamment du méthane, principal composant du gaz naturel.

La production du gaz naturel de synthèse donne la possibilité d'utiliser immédiatement toutes les infrastructures existantes développées pour cette énergie : réseaux de transport et de distribution, capacités de stockage, systèmes de production d'électricité, etc... En outre, il ressort également que le bilan carbone de cette production pourrait être nul, voire négatif, dès lors que l'électricité utilisée serait d'origine décarbonée et que le CO2 serait issu de systèmes utilisant des énergies fossiles que l'on aurait préalablement capté.

Pour réaliser l'électrolyse de l'eau (I), il est avantageux de la réaliser à haute température typiquement entre 600 et 950°C, car une partie de l'énergie nécessaire à la réaction peut être apportée par la chaleur qui est moins chère que l'électricité et l'activation de la réaction est plus efficace à haute température et ne nécessite pas de catalyseur. Pour mettre en oeuvre l'électrolyse à haute température, il est connu d'utiliser un électrolyseur de type SOEC (acronyme anglais de « *Solid Oxyde Electrolyte Cell* »), constitué d'un empilement de motifs élémentaires comportant chacun une cellule d'électrolyse à oxydes solides, constituée de trois couches anode/électrolyte/cathode superposées l'une sur l'autre, et de plaques d'interconnexion en alliages métalliques aussi appelées plaques bipolaires, ou interconnecteurs. Les interconnecteurs ont pour fonction d'assurer à la fois le passage du courant électrique et la circulation des gaz au voisinage de chaque cellule (vapeur d'eau injectée, hydrogène et oxygène extrait dans un électrolyseur EHT ; air et hydrogène injectés et eau extraite dans une pile SOFC) et de séparer les compartiments anodiques et cathodiques qui sont les compartiments de circulation des gaz du côté respectivement des anodes et des cathodes des cellules. Pour réaliser l'électrolyse de la vapeur d'eau à haute température EHT, on injecte de la vapeur d'eau H2O dans le compartiment cathodique. Sous l'effet du courant appliqué à la cellule, la dissociation des molécules d'eau sous forme vapeur est réalisée à l'interface entre l'électrode à hydrogène (cathode) et l'électrolyte : cette dissociation produit du gaz dihydrogène H2 et des ions oxygène. Le dihydrogène est collecté et évacué en sortie de compartiment à hydrogène. Les ions oxygène $O^{2-}$ migrent à travers l'électrolyte et se recombinent en dioxygène à l'interface entre l'électrolyte et l'électrode à oxygène (anode).

La co-électrolyse de la vapeur d'eau et du CO2 (II) offre potentiellement les mêmes avantages énergétiques et économiques que ceux décrits ci-dessus pour l'électrolyse de la vapeur d'eau (réaction (I)) sans l'inconvénient d'avoir à réaliser une condensation intermédiaire entre l'électrolyse de l'eau et l'électrolyse du CO2. Son intérêt réside dans la possibilité de réaliser la réaction de co-électrolyse (II) dans un même réacteur en maintenant celui-ci dans une gamme de températures voisines de 800°C. En effet à cette température, les tensions requises pour la réduction du CO2 en CO et de l'H2O en H2 sont quasi identiques. A titre d'exemple, les tensions à l'abandon, c'est-à-dire les tensions électriques obtenues sans courant électrique mais uniquement grâce aux différents gaz de part et d'autre d'une cellule, pour un mélange de 90% d'espèce oxydée et 10% d'espèce réduite à 800°C sont respectivement égales à 0,852V pour les couples H2O, H2/O2 et 0,844V pour les couples CO2, CO/O2.

De plus, la co-électrolyse à haute température présente le même intérêt énergétique que l'électrolyse de la vapeur d'eau entre 750 et 900°C par rapport à l'électrolyse de l'eau à basse température. En effet, l'énergie nécessaire à la dissociation des molécules H2O est diminuée de l'énergie de vaporisation. Par ailleurs, les cinétiques des réactions d'électrolyse de l'H2O et du CO2 sont fortement thermiquement activées et suivent une loi d'Arrhenius avec des énergies d'activation de l'ordre de 120kj/mol. Par conséquent, l'efficacité des réactions s'améliorent fortement lorsque la température est augmentée. La plus grande activité électrochimique à haute température permet en outre de s'affranchir de catalyseur onéreux, tel que le platine nécessaire à plus basses températures. En outre, la production de gaz de synthèse dans le compartiment cathodique du réacteur de co-électrolyse s'accompagne d'une production d'oxygène dans le compartiment anodique pouvant être valorisé par la suite, par exemple pour l'oxycombustion du gaz naturel.

Cela étant, si la co-électrolyse à haute température (II) telle qu'envisagée offre les avantages précités que sont l'investissement d'un unique réacteur d'électrolyse, le couplage thermique entre les différentes réactions, elle a pour inconvénient de ne pas permettre l'obtention de ratio H2/CO variable dans le gaz de mélange à la sortie du réacteur. Autrement dit, lorsqu'on réalise la co-électrolyse, un ratio H2/CO en sortie souhaitée impose un ratio H2O/CO2 donné en entrée. En effet, un fonctionnement proche du point de fonctionnement thermo-neutre fixe la tension à appliquer à l'électrolyseur. Ainsi, pour un ratio souhaité de H2/CO en sortie avec un taux de conversion de l'eau proche de 100%, les débits et compositions d'entrée en CO2 et H2O doivent nécessairement être déterminés.

Or, chaque gaz de synthèse destiné à produire un gaz combustible nécessite un ratio H2/CO donné en fonction du combustible visé. De même, la fabrication directe du gaz combustible nécessite un ratio CO2/H2 donné en fonction du combustible visé.

Le tableau 1 ci-dessous illustre ainsi les ratios nécessaires en fonction de procédés de synthèse de différents combustibles :

**TABLEAU 1**

| PROCEDE DE SYNTHESE | PRODUIT COMBUSTIBLE OBTENU | RATIO H2/CO NECESSAIRE | RATIO CO2/H2 NECESSAIRE |
|---|---|---|---|
| synthèse du méthane | Gaz naturel | 1/3 | 1/4 |
| synthèse du méthanol | méthanol | 1/2 | 1/3 |
| synthèse du diméthyléther(DME) | DME | 1/1 | 1/2 |
| synthèse Ficher Tropsch | Diesel | 1/2 | 1/3 |

La demanderesse a proposé dans la demande de brevet déposée le 17 décembre 2012 sous le numéro FR 12 62174 un nouveau procédé et réacteur de co-électrolyse permettant d'obtenir en sortie un ratio H2/CO variable et donc un gaz de synthèse dont la composition est adaptée pour produire le gaz combustible souhaité.

Par ailleurs, le point de fonctionnement retenu pour un réacteur d'électrolyse ou de co-électrolyse fixe aussi les conditions thermiques dans le réacteur d'électrolyse. En effet, pour les électrolyses réalisées à haute température, l'énergie $\Delta H$ nécessaire à la dissociation de la molécule d'entrée (H2O ou CO2) peut être apportée sous forme électrique et/ou de chaleur. L'énergie thermique apportée Q se définit alors en fonction de la tension U aux bornes de chaque cellule d'électrolyse par la relation :

$$Q = \frac{I}{2F}\Delta H - U \cdot I \, ,$$

dans laquelle U est la tension électrique, I est le courant électrique et F est la constante de Faraday. Ainsi, on définit trois régimes de fonctionnement, correspondant à trois modes thermiques différents pour l'empilement de cellules d'électrolyse :

- le mode dit autothermique dans lequel la tension imposée Uimp est égale à $\Delta H$ / 2F. La chaleur consommée par la réaction de dissociation est complètement compensée par les différentes résistances électriques de l'électrolyseur (irréversibilités). Le réacteur d'électrolyse (électrolyseur) n'a pas besoin de gestion thermique particulière tout en restant stable en température.
- le mode dit endothermique dans lequel la tension imposée Uimp est inférieure à $\Delta H$ / 2F. L'électrolyseur consomme plus de chaleur que les pertes électriques en son sein. Cette chaleur nécessaire doit alors lui être apportée par un autre moyen, sous peine de voir sa température chuter irrémédiablement.
- le mode dit exothermique dans lequel la tension imposée Uimp est supérieure à $\Delta H$ / 2F. L'électrolyse consomme alors moins de chaleur que les pertes électriques par effet Joule. Ce dégagement de chaleur au sein de l'électrolyseur doit alors être évacué par un autre moyen, sous peine de voir sa température augmenter de manière rédhibitoire.

Le mode endothermique nécessite une consommation moindre d'électricité : il y a donc peu de production et il faut fournir de la chaleur au réacteur d'électrolyse. L'intérêt de ce mode endothermique réside dans la disponibilité d'une source de chaleur peu coûteuse. Tout dépend alors de la nature et de la température de cette source thermique.

A contrario, le mode exothermique nécessite une consommation plus importante d'électricité : il y a donc une production importante, mais il faut refroidir le réacteur d'électrolyse, ce qui peut être très coûteux. L'intérêt de ce mode exothermique dépend alors beaucoup du coût de l'électricité et de l'utilisation de la chaleur excédentaire.

Ainsi, la gestion thermique d'un réacteur d'électrolyse ou de co-électrolyse est un facteur important à prendre en considération.

En outre, le transport, le stockage et l'utilisation de l'hydrogène nécessitent sa mise en pression. Il est déjà connu qu'au lieu de comprimer l'hydrogène produit, ce qui présente un coût important, de réaliser l'électrolyse de l'eau directement à partir de vapeur d'eau sous pression, l'eau étant alors comprimée à l'état liquide au préalable, ce qui est beaucoup moins coûteux.

Différents procédés d'obtention d'un gaz combustible par catalyse hétérogène soit directement à partir d'un mélange d'$H_2$ et dioxyde de carbone $CO_2$, soit indirectement à partir d'un gaz de synthèse ($H_2$ + CO) ont déjà été étudiés.

En particulier, l'hydrogénation de CO2 en méthane est un procédé industriel qui a été étudié à chaque choc énergétique, soit pour produire du méthane de synthèse à partir de $CO_2$ et $H_2$ purs, soit dans des centrales à gazéification du charbon avec des gaz et des conditions plus compliqués (procédé Fischer-Tropsch).

Pour le procédé de méthanation, deux voies sont possibles et ont été plus ou moins étudiées selon l'état de l'art.

La première voie est celle directe, avec une unique réaction selon l'équation suivante :

$$CO_2 + 4H_2 \rightarrow CH_4 + 2H_2O.$$

La deuxième voie est celle indirecte, avec une réaction en deux étapes selon les équations suivantes :

$$CO_2 + H_2 \rightarrow CO + H_2O.$$

$$CO + 3H_2 \rightarrow CH_4 + H_2O$$

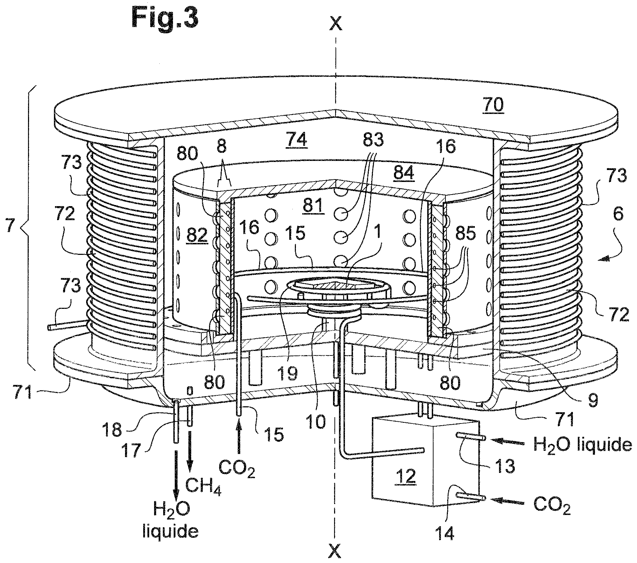

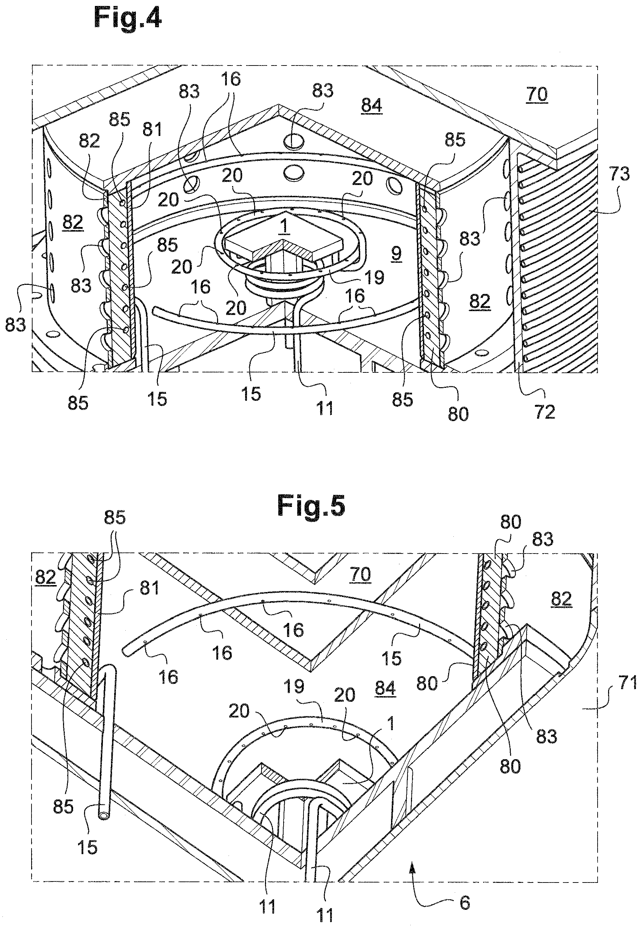

Comme mis en évidence par les auteurs de la publication [1] (voir notamment les figures 3 et 4), les réactions de méthanation sont favorisées à haute pression et à basse température selon la loi de Le Chatelier. En effet, les calculs thermodynamiques indiqués dans [1] indiquent une conversion théorique de 100% du CO2 en $CH_4$ à moins de 150°C contre 83% à 400°C. Cependant, il est également indiqué qu'une température minimum ainsi qu'une vitesse des gaz optimum est à ajuster pour garantir une cinétique suffisante. La température optimale à laquelle la méthanation doit être réalisée, est donc un compromis entre le taux de conversion souhaité du $CO_2$ et la cinétique recherchée de la réaction.

Les catalyseurs utilisés pour la méthanation sont en général à base de Nickel supporté par un oxyde de zirconium (ZrO2), ou à base de nickel (Ni) supporté par un oxyde d'aluminium ($Al_2O_3$). La publication [1] a mis en exergue l'activité catalytique importante pour un catalyseur à base de nickel (Ni) supporté par des oxydes mixtes de Cérium (Ce) et de zirconium de formule Ce0.72Zr0.28O2. De même, la publication [2] a montré, pour une méthanation sous pression de 30 bars, l'excellente activité catalytique d'un catalyseur bimétallique à base de nickel (Ni) et de fer (Fe) supporté par un oxyde d'aluminium ($Al_2O_3$) de formule Ni-Fe/$\gamma$- $Al_2O_3$.

Plusieurs types de réacteurs ont déjà été envisagés pour mettre en oeuvre la méthanation.

On peut citer tout d'abord les réacteurs à lit fixe dans lesquels le catalyseur solide est intégré sous forme de grains ou pastilles. L'inconvénient de ce type de réacteurs est la gestion thermique difficile à réaliser pour des réactions exothermiques comme la méthanation.

On peut citer également les réacteurs avec des canaux structurés tels que les réacteurs multitubulaires, les réacteurs monolithiques et les réacteurs à plaques, dans lesquels le catalyseur solide est généralement déposé sous forme de revêtement dans les canaux réactifs. Ces réacteurs sont bien adaptés pour une réaction de méthanation qui nécessite une bonne gestion thermique. Ils sont généralement plus onéreux.

Enfin, les réacteurs de type à lit fluidisé ou entrainé dans lesquels le catalyseur à fluidifier est sous forme de poudre. Ces réacteurs sont bien adaptés pour des réactions avec des volumes de réactifs très importants. De plus, la fluidisation du catalyseur permet une très bonne homogénéisation thermique du mélange de réactifs dans le réacteur et donc un meilleur contrôle thermique.

Quelle que soit la voie directe ou indirecte, le catalyseur solide, ou le type de réacteur utilisé à ce jour, la méthanation reste un procédé coûteux avec un rendement encore à améliorer, notamment du fait de la compression ultérieure du méthane obtenu qui est nécessaire à son stockage et/ou transport et du fait de la production séparée de l'hydrogène nécessaire, en particulier par électrolyse EHT ou co-électrolyse à haute température. Le couplage effectif à ce jour, entre méthanation et électrolyse, est loin d'avoir été réalisé.

La demande de brevet FR2931168 décrit un électrolyseur de type protonique, c'est-à-dire à circulation de protons H+ dans l'électrolyte, dans lequel on introduit à l'anode de l'eau $H_2O$ et on introduit $CO_2$ ou CO à la cathode, en vue de former du méthane ou d'autres combustibles. Le type de matériaux utilisés est loin d'être éprouvé. En outre, l'efficacité de la méthanation dans un tel électrolyseur protonique est loin d'avoir été prouvée.

Il existe donc un besoin pour améliorer le procédé de méthanation notamment en vue de baisser son coût d'investissement et de production et en vue d'améliorer son rendement.

Plus généralement, il existe un besoin pour améliorer les procédés de synthèse connus pour obtenir un gaz combustible choisi parmi le méthane, le méthanol ou le DME notamment en vue de baisser leurs coûts d'investissement et de production et en vue d'améliorer leurs rendements.

Le but de l'invention est de répondre au moins en partie à ces besoins.

Exposé de l'invention

Pour ce faire, l'invention concerne, sous l'un de ses aspects, et selon une première alternative un procédé d'obtention d'un gaz combustible choisi parmi le méthane, méthanol, le diméthyléther (DME) ou le diesel par catalyse hétérogène, comportant les étapes suivantes :

a/ une étape d'électrolyse à haute température de la vapeur d'eau $H_2O$ mise en oeuvre dans un réacteur d'électrolyse logé dans une enceinte étanche maintenue sous une pression donnée, étape a/ selon laquelle on alimente chaque cathode du réacteur en vapeur d'eau sous la pression donnée ;

b/ une étape de conversion catalytique mise en oeuvre au sein d'au moins une zone de réaction agencée à distance et radialement au réacteur d'électrolyse dans la même enceinte sous pression et contenant au moins un catalyseur solide de la conversion, l'étape b/ étant réalisée à partir de l'hydrogène H2 produit lors de l'étape a/ d'électrolyse et, de dioxyde de carbone $CO_2$ injecté dans l'espace entre le réacteur d'électrolyse et la zone de réaction radiale ;

c/ une étape de récupération du gaz combustible produit et de la vapeur d'eau non convertie selon l'étape a/ et produite selon l'étape b/, dans l'espace entre ladite zone de réaction radiale et la(les) paroi(s) délimitant l'enceinte.

Selon une deuxième alternative, l'invention concerne un procédé d'obtention d'un gaz combustible choisi parmi le méthane, méthanol, le diméthyléther (DME) ou le diesel par catalyse hétérogène, comportant les étapes suivantes :

a'/ une étape de co-électrolyse à haute température de la vapeur d'eau $H_2O$ et du dioxyde de carbone $CO_2$ mise en oeuvre dans un réacteur de co-électrolyse logé dans une enceinte étanche maintenue sous une pression donnée ; étape a'/ selon laquelle on alimente chaque cathode du réacteur en vapeur d'eau $H_2O$ et en dioxyde de carbone $CO_2$ sous la pression donnée ;

b'/ une étape de conversion catalytique étant mise en oeuvre au sein d'au moins une zone de réaction agencée à distance et radialement au réacteur de co-électrolyse dans la même enceinte sous pression et contenant au moins un catalyseur solide de la conversion, l'étape b'/ étant réalisée à partir de l'hydrogène $H_2$ et du monoxyde de carbone CO produits lors de l'étape a'/ de co-électrolyse ;

c'/ une étape de récupération du gaz combustible produit et de la vapeur d'eau non convertie selon l'étape a'/ et produite selon l'étape b'/, dans l'espace entre ladite zone de réaction radiale et la(les) paroi(s) délimitant l'enceinte.

On précise que dans le contexte de l'invention, les hautes températures de l'étape a) d'électrolyse ou a') de co-électrolyse ne peuvent être confondues avec les températures basses auxquelles une électrolyse de type alcaline est réalisée.

Par « enceinte étanche sous pression donnée », il faut comprendre ici et dans le cadre de l'invention une enceinte étanche vis-à-vis de l'atmosphère extérieure et dont l'intérieur est maintenu à une pression supérieure à la pression atmosphérique.

Selon un mode de réalisation avantageux, la zone de réaction est constituée par une cloison poreuse contenant le catalyseur solide de la conversion.

Par « cloison poreuse », on entend un ensemble formé d'une ou plusieurs parois dont la porosité globale laisse passer les gaz présents dans l'enceinte, i.e. le méthane formé dans la cloison et la vapeur d'eau. L'ensemble peut ainsi être constitué d'au moins deux grilles, grillages, tôles métalliques ou deux substrats en céramique très poreuse et dont l'espace les séparant contient au moins un catalyseur solide de la conversion selon l'étape b/ ou b'/.

Par « zone de réaction agencée à distance » et « cloison poreuse agencée à distance », on entend un agencement avec un espacement suffisant entre la zone (cloison poreuse et le réacteur d'électrolyse/co-électrolyse pour que la température des gaz ait atteint une gamme de valeurs appropriées à la mise en oeuvre de l'étape b) ou b'). Typiquement, la température optimale de mise en oeuvre de l'étape b) ou b') de méthanation est de l'ordre de 400°C, on s'assure ainsi d'un espacement suffisant pour que l'$H_2$ produit avec le $CO_2$ injecté ou le mélange $H_2$+CO produit aux environs de 800 à 850°C ait atteint une température de l'ordre de 400°C lorsqu'il pénètre dans la zone de réaction (cloison poreuse).

L'étape b/ ou b'/ est de préférence réalisée avec la zone de réaction radiale fermée sur elle-même en étant agencée de manière concentrique autour du réacteur d'électrolyse ou respectivement de co-électrolyse.

L'étape a/ ou a'/ est avantageusement réalisée à des températures comprises entre 600°C et 1000°C, de préférence entre 650°C et 850°C ; de préférence encore entre 700 et 800°C

L'étape b/ ou b'/ est avantageusement réalisée à des températures comprises entre 250°C et 500°C, de préférence entre 300°C et 400°C.

L'étape a/ ou a'/ est de préférence réalisée à des pressions comprises entre 0 et 100 bars, de préférence comprise entre 4 et 80 bars, c'est-à-dire une gamme comprise entre la pression dans un réseau de distribution moyenne pression (4 bars) et celle dans les gazoducs de transport (80 bars)

Selon un mode de réalisation avantageux, on refroidit les parois délimitant l'enceinte à une température inférieure à la température de saturation de l'eau à la pression donnée de l'enceinte, de sorte que l'étape c/ ou c'/ consiste en une séparation du gaz combustible de l'eau condensée au sein de l'enceinte, puis une récupération du gaz combustible séparé et de l'eau condensée par gravité sur le fond de l'enceinte.

Le procédé constitue avantageusement un procédé de méthanation. Dans un tel procédé, avantageusement, la pression donnée de l'enceinte et de fonctionnement du réacteur d'électrolyse ou de co-électrolyse est égale à environ 30 bars, la température de mise en oeuvre de l'étape a/ ou a'/ étant maintenue égale à environ 800°C, la température au sein de la zone de réaction radiale étant maintenue égale à environ 400°C, la température des parois délimitant l'enceinte étant maintenue inférieure à 230°C.

L'invention concerne également sous un autre de ses aspects, un réacteur pour l'obtention d'un gaz combustible choisi parmi le méthane, méthanol, le diméthyléther (DME) ou le diesel par catalyse hétérogène, comportant :

- une enceinte étanche apte à être mise sous pression donnée;
- un réacteur soit d'électrolyse à haute température de la vapeur d'eau soit de co-électrolyse à haute températures de vapeur d'eau et de dioxyde de carbone, comportant un empilement de cellules d'électrolyse élémentaires de type SOEC formées chacune d'une cathode, d'une anode et d'un électrolyte intercalé entre la cathode et l'anode, et une pluralité d'interconnecteurs électrique et fluidique agencés chacun entre deux cellules élémentaires adjacentes avec une de ses faces en contact électrique avec l'anode d'une des deux cellules élémentaires et l'autre de ses faces en contact électrique avec la cathode de l'autre des deux cellules élémentaires, le réacteur d'électrolyse ou de co-électrolyse étant logé dans l'enceinte et la sortie des cathodes étant débouchante à l'intérieur de l'enceinte;
- au moins une cloison poreuse agencée à distance et radialement au réacteur d'électrolyse ou de co-électrolyse dans l'enceinte et contenant au moins un catalyseur solide de conversion de gaz de synthèse ($H_2$+CO ou $H_2$+$CO_2$) en gaz combustible ;
- au moins un tuyau d'alimentation en vapeur d'eau sous pression et, le cas échéant en dioxyde de carbone, des cathodes du réacteur d'électrolyse ou de co-électrolyse,
- le cas échéant, au moins un tuyau d'injection en dioxyde de carbone de l'espace entre le réacteur d'électrolyse et la cloison poreuse ;
- au moins un tuyau de récupération de gaz combustible et/ou de vapeur d'eau,
- le cas échéant, au moins un tuyau de récupération d'eau condensée sur les parois délimitant l'enceinte, chaque tuyau traversant une paroi délimitant l'enceinte.

On précise ici que, les dispositifs d'interconnexion, électrique et fluidique, aussi appelés interconnecteurs ou encore plaques d'interconnexion, sont les dispositifs qui assurent la connexion en série d'un point de vue électrique de chaque cellule d'électrolyse dans l'empilement des réacteurs EHT et en parallèle d'un point de vue fluidique, combinant ainsi la production de chacune des cellules. Les interconnecteurs assurent ainsi les fonctions d'amenée et de collecte de courant et délimitent des compartiments de circulation (distribution et/ou la collecte) des gaz.

Les cellules d'électrolyse sont avantageusement de type à cathode support. Par «cellule à cathode support », on entend ici et dans le cadre de l'invention la définition déjà donnée dans le domaine de l'électrolyse de l'eau à haute température EHT et désignée sous l'acronyme anglais CSC pour « *Cathode-supported Cell* », c'est-à-dire une cellule dans laquelle l'électrolyte et l'électrode à oxygène (anode) sont disposées sur l'électrode à hydrogène ou à monoxyde de carbone (cathode) plus épaisse qui sert donc de support.

Selon un mode de réalisation avantageux, la cloison poreuse est fermée sur elle-même en étant agencée de manière concentrique autour du réacteur d'électrolyse ou de co-électrolyse.

La cloison poreuse est de préférence constituée de deux parois métalliques poreuses, l'espace les séparant étant rempli au moins partiellement d'un catalyseur de conversion sous forme de poudre ou de granulats. Les deux parois métalliques sont de préférence chacune constituée d'une tôle perforée d'une pluralité de trous régulièrement espacés à la fois sur la hauteur et sur la longueur de la cloison.

Le catalyseur solide de conversion est de préférence à base de nickel (Ni) supporté par un oxyde de zirconium (ZrO2), ou à base de nickel (Ni) supporté par un oxyde d'aluminium ($Al_2O_3$), ou bimétallique à base de nickel (Ni) et de fer (Fe) supporté par un oxyde d'aluminium ($Al_2O_3$), de préférence Ni-Fe/$\gamma$- $Al_2O_3$, ou à base de nickel (Ni) supporté par des oxydes mixtes de Cérium (Ce) et de zirconium, de préférence $Ce_{0.72}Zr_{0.28}O_2$.

La cloison poreuse comporte avantageusement, au sein du catalyseur solide, une partie de circuit de refroidissement apte à refroidir la réaction catalytique entre l'hydrogène et le monoxyde de carbone produits en amont dans le réacteur de co-électrolyse ou entre l'hydrogène produit en amont dans le réacteur d'électrolyse et du dioxyde de carbone injecté dans l'espace entre la cloison poreuse et le réacteur d'électrolyse.

Le tuyau d'alimentation est de préférence enroulé en partie sur lui-même à proximité du réacteur d'électrolyse ou de co-électrolyse pour réchauffer la vapeur d'eau sous pression et le cas échéant le dioxyde de carbone avant d'alimenter les cathodes.

Selon une variante de réalisation avantageuse, le réacteur comporte un tuyau de récupération de l'hydrogène et le cas échéant du monoxyde de carbone produit(s) aux cathodes, le tuyau de récupération étant enroulé sur lui-même en formant un cercle et étant percé d'une pluralité de trous répartis régulièrement le long du cercle pour diffuser de manière homogène l'hydrogène et le cas échéant du monoxyde de carbone dans l'espace entre le réacteur d'électrolyse ou de co-électrolyse et la cloison poreuse agencée de manière concentrique.

Le tuyau d'injection du dioxyde de carbone est de préférence enroulé sur lui-même en formant un cercle et étant percé d'une pluralité de trous répartis régulièrement le long du cercle pour diffuser de manière homogène le dioxyde de carbone dans l'espace entre le réacteur d'électrolyse ou de co-électrolyse et la cloison poreuse agencée de manière concentrique.

Selon une variante de réalisation avantageuse, l'enceinte étanche comporte une enveloppe latérale, un couvercle et un fond assemblés à l'enveloppe de manière étanche, et un premier support pour supporter à la fois le réacteur d'électrolyse ou de co-électrolyse et de la cloison poreuse afin de les agencer à distance du fond et du couvercle de l'enceinte.

De préférence, le réacteur comporte un deuxième support, fixé sur le premier support, pour supporter uniquement le réacteur d'électrolyse ou de co-électrolyse afin de l'agencer en regard de la portion centrale de la cloison poreuse, de préférence à mi-hauteur de la cloison poreuse.

Selon une variante de réalisation avantageuse, l'enveloppe latérale comporte une partie d'un circuit de refroidissement à une température inférieure à la température de saturation de l'eau sous la pression donnée.

Le fond de l'enceinte étanche constitue avantageusement une cuvette de récupération de l'eau condensée sur le couvercle et/ou l'enveloppe latérale et/ou le fond.

L'invention concerne sous encore un autre de ses aspects, un système comportant :

- un réacteur qui vient d'être décrit;
- un échangeur de chaleur formant un générateur de vapeur pour vaporiser de l'eau liquide sous la pression donnée, l'échangeur étant agencé à l'extérieur de l'enceinte.

Dans un tel système, une partie du circuit secondaire de l'échangeur comporte avantageusement le tuyau de récupération de l'eau condensée dans le fond.

Le circuit de refroidissement de la cloison poreuse constitue avantageusement le circuit primaire de l'échangeur de chaleur pour vaporiser l'eau liquide sous la pression donnée.

L'invention concerne sous encore un autre de ses aspects, un procédé de fonctionnement d'un réacteur de co-électrolyse décrit ci-dessus, selon lequel on alimente et on distribue la vapeur d'eau à la cathode d'une des deux cellules élémentaires adjacentes et on alimente et on distribue du dioxyde de carbone à la cathode de l'autre des deux cellules élémentaires.

Selon un mode de réalisation avantageux, on définit un régime de fonctionnement en mode exothermique pour l'électrolyse de la vapeur d'eau à la cathode d'une des deux cellules élémentaires adjacentes et simultanément on réalise un régime de fonctionnement en mode endothermique pour l'électrolyse du dioxyde de carbone à la cathode de l'autre des deux cellules élémentaires adjacentes, la chaleur dégagée par l'électrolyse de la vapeur d'eau étant apte à apporter au moins en partie la chaleur requise par l'électrolyse du dioxyde de carbone.

Alternativement, on définit un régime de fonctionnement en mode exothermique pour l'électrolyse du dioxyde de carbone à la cathode d'une des deux cellules élémentaires adjacentes et simultanément on réalise un régime de fonctionnement en mode endothermique pour l'électrolyse de la vapeur d'eau de l'autre des deux cellules élémentaires adjacentes, la chaleur dégagée par l'électrolyse du dioxyde de carbone étant apte à apporter au moins en partie la chaleur requise par l'électrolyse de la vapeur d'eau.

L'invention concerne en outre l'utilisation du réacteur décrit ou du système décrit en tant que réacteur de méthanation.

L'invention concerne en outre l'utilisation du réacteur décrit en tant que que réacteur de reformage catalytique et de pile à combustible, l'enceinte n'étant pas sous pression, le tuyau de récupération du gaz combustible constituant un tuyau d'alimentation en gaz combustible et le réacteur d'électrolyse ou de co-électrolyse à empilement de cellules constituant une pile à combustible SOFC.

Autrement dit, les procédés de conversion selon l'invention, en particulier de méthanation consistent essentiellement à injecter de la vapeur d'eau sous pression dans une enceinte, à électrolyser la vapeur d'eau $H_2O$ ou à co-électrolyser la vapeur d'eau $H_2O$ et le dioxyde de carbone $CO_2$ à haute température et à réaliser la conversion catalytique en gaz combustible au sein de la même enceinte maintenue sous pression, en agençant la zone de réaction à une distance suffisante du réacteur d'électrolyse ou de co-électrolyse pour obtenir une gamme de températures des gaz optimale pour la conversion catalytique. Le procédé selon l'invention est mis en oeuvre avantageusement grâce au réacteur selon l'invention.

Autrement dit, l'invention permet de produire du méthane à une pression de l'électrolyse de l'eau à haute température déjà éprouvée, typiquement 30 bars, sans avoir à investir spécifiquement dans un ou plusieurs équipements dédiés à la mise en pression puisque l'enceinte étanche sous pression selon l'invention sert à la fois comme enceinte pour la conversion catalytique et pour l'électrolyse/co-électrolyse.

On peut avantageusement réaliser la co-électrolyse de vapeur d'eau et du dioxyde de carbone dans le réacteur à empilement selon l'enseignement de la demande FR 12 62174 précitée : on alimente et on distribue la vapeur d'eau à la cathode d'une des deux cellules élémentaires adjacentes et on alimente et on distribue du dioxyde de carbone à la cathode de l'autre des deux cellules élémentaires. Cela permet de faire varier à souhait le ratio $H_2/CO$ obtenu en sortie avant son mélange pour constituer le gaz de synthèse converti en gaz combustible dans l'enceinte, et de faciliter la gestion thermique de l'empilement des cellules d'électrolyse quel que soit le mode de fonctionnement (mode endothermique ou exothermique), et ce, de manière réversible en fonction du coût du courant.

Les avantages de l'électrolyse de vapeur d'eau sous pression ou de co-électrolyse de la vapeur d'eau et du dioxyde de carbone combinée à une conversion catalytique en gaz combustible au sein d'une même enceinte maintenue sous pression, conformément à l'invention sont nombreux. Parmi ceux-ci, on peut citer:

- mise en oeuvre d'un unique appareil avec une seule enceinte pour réaliser à la fois l'électrolyse de la vapeur d'eau ou la co-électrolyse de vapeur d'eau et de CO2 et de la conversion catalytique en gaz combustible, plus particulièrement la méthanation, ce qui permet de de limiter l'investissement ;
- forte intégration de la gestion thermique entre électrolyse/co-électrolyse et conversion catalytique au sein de la même enceinte comparativement aux procèdes connus nécessitant la mise en oeuvre séquentielle d'au moins deux réacteurs différents ;
- dimensionnement de la résistance à la pression pour une seule enceinte à la fois pour l'électrolyse/co-électrolyse et pour la conversion catalytique (méthanation). En particulier, la conversion catalytique peut se faire à pression élevée requise pour l'electrolyse/co-électrolyse sans nécessité d'investir dans une enceinte supplémentaire. La ou les parois constituant la cloison poreuse agencée à distance du réacteur d'électrolyse ou de co-électrolyse, pour la réalisation de l'étape b/ ou b'/, en particulier la méthanation peuvent être de conception très simples et à bas coût.
- réalisation de la conversion catalytique, en particulier la méthanation, sous pression, ce qui permet un fonctionnement du catalyseur solide sur une large plage de températures et donc introduit une certaine souplesse dans la gestion thermique. Cela permet aussi de faire la conversion catalytique à forte pression, typiquement la pression usuellement rencontrée dans les gazoducs de transport de méthane, i.e. à 80bars, sans avoir d'investissement spécifique à faire. En particulier, on peut s'affranchir de toute compression du gaz combustible, tel que le méthane $CH_4$ en sortie de l'enceinte selon l'invention ;
- envoi direct du méthane obtenu dans le réseau de gaz sous pression s'il reste moins de 10% d'hydrogène non transformé ;
- suppression possible de tout gradient thermique néfaste au sein de la cloison poreuse, grâce à l'agencement concentrique possible de la cloison poreuse fermée sur elle-même autour et à distance du réacteur d'électrolyse/co-électrolyse, le trajet du gaz à convertir au sein du catalyseur peut être relativement court, même pour une grande quantité de catalyseur, ce qui est favorable pour la gestion thermique de la conversion catalytique, telle que la méthanation qui a lieu sur toute la circonférence de la cloison. L'épaisseur de la cloison contenant le catalyseur solide peut alors être relativement faible devant ses autres dimensions ;
- meilleure gestion des risques liés à l'utilisation de l'enceinte sous pression comparativement aux électrolyseurs EHT selon l'état de l'art, du fait d'une part de la diminution du volume de gaz requis pour le même gradient thermique entre le réacteur d'électrolyse et les parois délimitant l'enceinte et d'autre part, de la fonction d'écran thermique à moindre température conférée à la cloison poreuse, typiquement à 400°C pour la méthanation, vis-à-vis des parois de l'enceinte dont on veut maîtriser la température ;
- souplesse supplémentaire pour la gestion thermique du réacteur globale selon l'invention grâce à l'échauffement provoqué par l'introduction du gaz de synthèse en paroi interne de la cloison poreuse et qui est localisé dans un espace déjà chaud de l'enceinte ;
- flexibilité d'utilisation du réacteur selon l'invention puisque d'une part, il est possible de réaliser la méthanation soit par voie directe soit par voie indirecte en injectant du $CO_2$ et d'autre part, on peut inverser son fonctionnement en injectant du méthane $CH_4$, la cloison contenant le catalyseur solide fonctionnant alors comme pré-reformeur catalytique et le réacteur d'électrolyse de type SOEC fonctionnant comme une pile à combustible SOFC ; autrement dit, dans le cadre de l'invention, l'inversion conduit à utiliser la cloison avec le catalyseur solide en reformeur et le réacteur d'électrolyse SOEC en pile à combustible SOFC de manière à produire du courant électrique ;
- moindre consommation d'eau et moindre investissement d'équipement de traitement de l'eau comparativement à une électrolyse EHT et une méthanation séquentielles selon l'état de l'art. Ainsi, le maintien des parois délimitant l'enceinte à une température inférieure à la température de saturation de l'eau à la pression donnée permet de pouvoir séparer le méthane produit et l'eau non transformée en condensant cette dernière sur lesdites parois. L'eau ainsi condensée peut alors être réinjectée dans le dispositif de production de vapeur du système (échangeur de chaleur de vaporisation). Par conséquent, comparativement à un système selon l'état de l'art à réacteur de méthanation et réacteur d'électrolyse de l'eau séparés, on évite l'investissement d'un condenseur en pression pour obtenir du méthane sec ;
- selon l'application visée pour l'utilisation du méthane obtenu selon l'invention, si la pression de l'enceinte et donc du méthane obtenu est trop élevée, possibilité de détendre le méthane et par là de participer au refroidissement de l'enceinte ;

- possibilité de liquéfier le méthane sous très forte pression obtenu selon l'invention par détentes successives pour son transport.

Description détaillée

D'autres avantages et caractéristiques de l'invention ressortiront mieux à la lecture de la description détaillée d'exemples de mise en oeuvre de l'invention faite à titre illustratif et non limitatif en référence aux figures suivantes parmi lesquelles :

- la figure 1 est une vue schématique montrant le principe de fonctionnement d'un électrolyseur d'eau à haute température;
- la figure 2 est une vue schématique en éclaté d'une partie d'un électrolyseur de vapeur d'eau à haute température comprenant des interconnecteurs,
- la figure 3 est une vue en perspective partiellement arrachée d'un réacteur selon l'invention mettant en oeuvre dans une même enceinte sous pression soit une électrolyse à haute température de la vapeur d'eau $H_2O$ soit une co-électrolyse à haute température de la vapeur d'eau $H_2O$ et du dioxyde de carbone $CO_2$ et une méthanation à partir du (des) gaz produit(s) par l'électrolyse ou la co-électrolyse,
- la figure 4 est une vue de détail en perspective partiellement écorchée du réacteur selon la figure 3,
- la figure 5 est une autre vue de détail en perspective partiellement écorchée du réacteur selon la figure 3.

Dans l'ensemble de la présente demande, les termes « vertical », « inférieur », « supérieur », « bas », « haut », « dessous » et « dessus » sont à comprendre par référence par rapport à un réacteur d'obtention d'un gaz combustible avec son enceinte sous pression tels qu'ils sont en configuration verticale de fonctionnement. Ainsi, dans une configuration de fonctionnement, l'enceinte est agencée à la verticale avec son fond vers le bas et le réacteur d'électrolyse ou de co-électrolyse est agencé avec ses cellules à l'horizontale sur son support dédié.

De même, dans l'ensemble de la présente demande, les termes « entrée », « sortie » « aval » et « amont » sont à comprendre en référence au sens de circulation des gaz depuis leur entrée dans le réacteur d'électrolyse EHT ou de co-électrolyse ou dans l'enceinte étanche sous pression jusqu'à leur sortie de celle-ci.

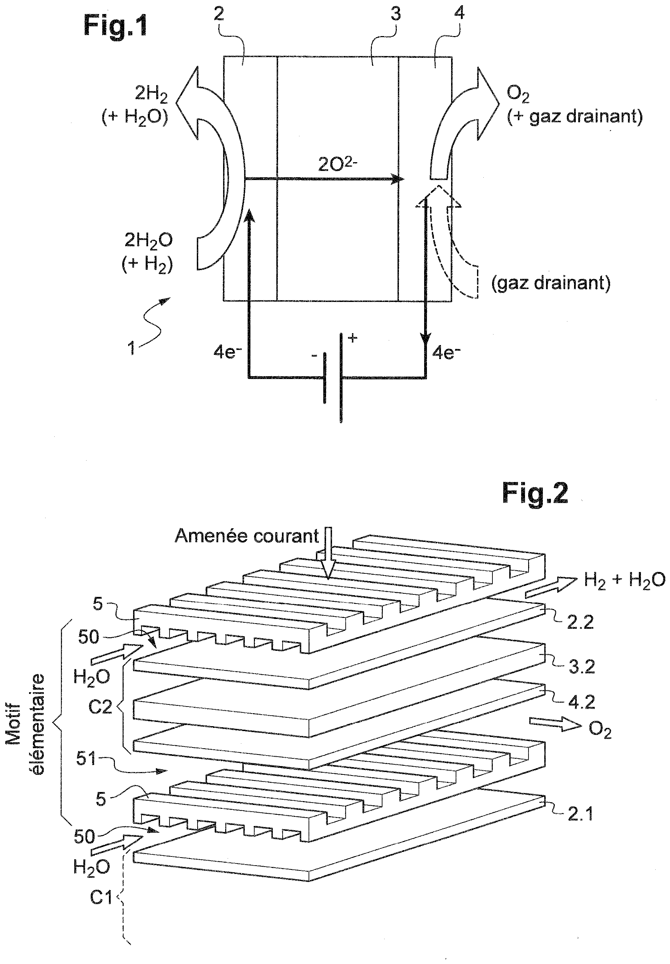

On précise que sur l'ensemble des figures 1 à 5, les symboles et les flèches d'alimentation de vapeur d'eau $H_2O$, de distribution et de récupération de dihydrogène $H_2$ et d'oxygène $O_2$, et du courant, de dioxyde de carbone $CO_2$, de distribution et de récupération de monoxyde de carbone CO et d'oxygène $O_2$ et du courant, et du méthane $CH_4$ sont montrés à des fins de clarté et de précision, pour illustrer le fonctionnement d'un réacteur d'électrolyse de vapeur d'eau ou de co-électrolyse simultanée de vapeur d'eau et de dioxyde de carbone connus et d'un réacteur de méthanation selon l'invention.

On précise également que sur les figures 3 à 5 relatives à un réacteur de méthanation selon l'invention, la récupération d'oxygène $O_2$ en sortie de l'électrolyseur ou du co-électrolyseur n'est pas représentée à des fins de clarté.

On précise également que tous les électrolyseurs ou co-électrolyseurs décrits sont de type à oxydes solides (SOEC, acronyme anglais de « *Solid Oxide Electrolyte Cell* ») fonctionnant à haute température. Ainsi, tous les constituants (anode/électrolyte/cathode) d'une cellule d'électrolyse sont des céramiques.

De tels constituants peuvent être ceux d'une pile à combustible SOFC. La haute température de fonctionnement d'un électrolyseur (réacteur d'électrolyse) est typiquement comprise entre 600°C et 1000°C. De préférence, dans le cadre de l'invention, on envisage une gamme préférée entre 650 et 850°C et de préférence encore entre 700 et 800°C.

Typiquement, les caractéristiques d'une cellule d'électrolyse élémentaire SOEC convenant à l'invention, du type cathode support (CSC), peuvent être celles indiquées comme suit dans le tableau 2 ci-dessous.

TABLEAU 2

| Cellule d'électrolyse | Unité | Valeur |
|---|---|---|
| **Cathode 2** | | |
| Matériau constitutif | | Ni-YSZ |
| Epaisseur | $\mu$m | 315 |
| Conductivité thermique | W m$^{-1}$ K$^{-1}$ | 13,1 |
| Conductivité électrique | $\Omega^{-1}$ m$^{-1}$ | $10^5$ |
| Porosité | | 0,37 |
| Perméabilité | m$^2$ | $10^{-13}$ |

(suite)

| Cellule d'électrolyse | Unité | Valeur |
|---|---|---|
| **Cathode 2** | | |
| Tortuosité | | 4 |
| Densité de courant | A.m$^{-2}$ | 5300 |
| **Anode 4** | | |
| Matériau constitutif | | LSM |
| Epaisseur | $\mu$m | 20 |
| Conductivité thermique | W m$^{-1}$ K$^{-1}$ | 9,6 |
| Conductivité électrique | $\Omega^{-1}$ m$^{-1}$ | 1 10$^4$ |
| Porosité | | 0,37 |
| Perméabilité | m$^2$ | 10$^{-13}$ |
| Tortuosité | | 4 |
| Densité de courant | A.m$^{-2}$ | 2000 |
| **Electrolyte 3** | | |
| Matériau constitutif | | YSZ |
| Epaisseur | $\mu$m | |
| Résistivité | $\Omega$ m | 0,42 |

Un électrolyseur d'eau est un dispositif électrochimique de production d'hydrogène (et d'oxygène) sous l'effet d'un courant électrique.

Dans les électrolyseurs à haute température EHT, l'électrolyse de l'eau à haute température est réalisée à partir de vapeur d'eau. La fonction d'un électrolyseur haute température EHT est de transformer la vapeur d'eau en hydrogène et en oxygène selon la réaction suivante:

$$2H_2O \rightarrow 2H_2 + O_2.$$

Cette réaction est réalisée par voie électrochimique dans les cellules de l'électrolyseur. Comme schématisée en figure 1, chaque cellule d'électrolyse élémentaire 1 est formée d'une cathode 2 et d'une anode 4, placées de part et d'autre d'un électrolyte solide 3. Les deux électrodes (cathode et anode) 2,4 sont des conducteurs électroniques, en matériau poreux, et l'électrolyte 3 est étanche au gaz, isolant électronique et conducteur ionique. L'électrolyte peut être en particulier un conducteur anionique, plus précisément un conducteur anionique des ions $O^{2-}$ et l'électrolyseur est alors dénommé électrolyseur anionique.

Les réactions électrochimiques se font à l'interface entre chacun des conducteurs électroniques et le conducteur ionique.

A la cathode 2, la demi-réaction est la suivante :

$$2 H2O \rightarrow + 4e^- \rightarrow 2 H2 + 2 O^{2-}.$$

A l'anode 4, la demi-réaction est la suivante:

$$2O^{2-} \rightarrow O2 + 4 e^-.$$

L'électrolyte 3 intercalé entre les deux électrodes 2, 4 est le lieu de migration des ions $O^{2-}$, sous l'effet du champ électrique créé par la différence de potentiel imposée entre l'anode 4 et la cathode 2.

Comme illustré entre parenthèses en figure 1, la vapeur d'eau en entrée de cathode peut être accompagnée d'hydrogène $H_2$ et l'hydrogène produit et récupéré en sortie peut être accompagné de vapeur d'eau. De même, comme illustré en pointillés, un gaz drainant, tel que l'air peut en outre être injecté en entrée pour évacuer l'oxygène produit. L'injection d'un gaz drainant a pour fonction supplémentaire de jouer le rôle de régulateur thermique.

Un réacteur d'électrolyse élémentaire est constitué d'une cellule élémentaire telle que décrite ci-dessus, avec une cathode 2, un électrolyte 3, et une anode 4 et de deux connecteurs mono-polaires qui assurent les fonctions de distribution électrique, hydraulique et thermique.

Pour augmenter les débits d'hydrogène et d'oxygène produits, il est connu d'empiler plusieurs cellules d'électrolyse élémentaires les unes sur les autres en les séparant par des dispositifs d'interconnexion, usuellement appelés interconnecteurs ou plaques d'interconnexion bipolaires. L'ensemble est positionné entre deux plaques d'interconnexion d'extrémité qui supportent les alimentations électriques et des alimentations en gaz de l'électrolyseur (réacteur d'électrolyse).

Un électrolyseur de l'eau à haute température (EHT) comprend ainsi au moins une, généralement une pluralité de cellules d'électrolyse empilées les uns sur les autres, chaque cellule élémentaire étant formée d'un électrolyte, d'une cathode et d'une anode, l'électrolyte étant intercalé entre l'anode et la cathode.

Les dispositifs d'interconnexion fluidique et électrique qui sont en contact électrique avec une ou des électrodes assurent en général les fonctions d'amenée et de collecte de courant électrique et délimitent un ou des compartiments de circulation des gaz.

Ainsi, un compartiment dit cathodique a pour fonction la distribution du courant électrique et de la vapeur d'eau ainsi que la récupération de l'hydrogène à la cathode en contact.

Un compartiment dit anodique a pour fonction la distribution du courant électrique ainsi que la récupération de l'oxygène produit à l'anode en contact, éventuellement à l'aide d'un gaz drainant.

Un fonctionnement satisfaisant d'un électrolyseur EHT nécessite:

- une bonne isolation électrique entre deux interconnecteurs adjacents dans l'empilement, sous peine de court-circuiter la cellule d'électrolyse élémentaire intercalée entre les deux interconnecteurs,
- un bon contact électrique et une surface de contact suffisante entre chaque cellule et interconnecteur, afin d'obtenir la plus faible résistance ohmique entre cellule et interconnecteurs,
- une bonne étanchéité entre les deux compartiments distincts, i.e. et cathodique, sous peine de recombinaison des gaz produits entraînant une baisse de rendement et surtout l'apparition de points chauds endommageant l'électrolyseur,
- une bonne distribution des gaz à la fois en entrée et en récupération des gaz produits, sous peine de perte de rendement, d'inhomogénéité de pression et de température au sein des différentes cellules élémentaires voire de dégradations rédhibitoires des cellules.

La figure 2 représente une vue éclatée de motifs élémentaires d'un électrolyseur de vapeur d'eau à haute température selon l'état de l'art. Cet électrolyseur EHT comporte une pluralité de cellules d'électrolyse élémentaires C1, C2, de type à oxyde solide (SOEC) empilées alternativement avec des interconnecteurs 5 Chaque cellule C1, C2... est constituée d'une cathode 2.1, 2.2,... et d'une anode 4.1, 4.2, entre lesquelles est disposé un électrolyte 3.1, 3.2....

L'interconnecteur 5 est un composant en alliage métallique qui assure la séparation entre les compartiments cathodique 50 et anodique 51, définis par les volumes compris entre i'interconnecteur 5 et l'anode adjacente 4.2 et entre l'interconnecteur 5 et la cathode adjacente 2.1 respectivement. Il assure également la distribution des gaz aux cellules. L'injection de vapeur d'eau dans chaque motif élémentaire se fait dans le compartiment cathodique 50. Le collectage de l'hydrogène produit et de la vapeur d'eau résiduelle à la cathode 2.1, 2.2 ... est effectué dans le compartiment cathodique 50 en aval de la cellule C1 C2... après dissociation de la vapeur d'eau par celle-ci. Le collectage de l'oxygène produit à l'anode 4.2 est effectué dans le compartiment anodique 51 en aval de la cellule C1 C2... après dissociation de la vapeur d'eau par celle-ci.

L'interconnecteur 5 assure le passage du courant entre les cellules C1 et C2 par contact direct avec les électrodes adjacentes, c'est-à-dire entre l'anode 4.2 et la cathode 2.1.

Dans les co-électrolyseurs à haute température EHT, la co-électrolyse à haute température est réalisée à partir de vapeur d'eau et de dioxyde de carbone $CO_2$. La fonction d'un co-électrolyseur haute température SOEC est de transformer la vapeur d'eau et le $CO_2$ en hydrogène, monoxyde de carbone et en oxygène selon la réaction suivante:

$$CO_2 + H_2O \rightarrow CO + H_2 + O_2.$$

Un co-électrolyseur 1 peut comporter exactement les mêmes constituants à oxydes solides (SOEC) qu'un électrolyseur EHT qui vient d'être décrit. Usuellement, la vapeur d'eau et le dioxyde de carbone $CO_2$ sont mélangés avant l'entrée dans le co-électrolyseur et injectés simultanément dans chaque compartiment cathodique 50.

Afin d'obtenir un ratio variable entre les gaz de sortie produits $H_2/CO$, et ce quel que soit le mode de fonctionnement exothermique ou endothermique d'une cellule d'électrolyse donnée, la demanderesse a proposé, dans la demande de brevet FR 12 62174 précitée, un nouveau procédé d'électrolyse simultanée mais séparée de la vapeur d'eau et du $CO_2$.

Plus précisément, le procédé de co-électrolyse à haute température de la vapeur d'eau H$_2$O et du dioxyde de carbone CO$_2$ selon la demande de brevet FR 12 62174 est mis en oeuvre par le réacteur d'electrolyse comportant un empilement de cellules d'électrolyse élémentaires de type SOEC (C1, C2, C3) formées chacune d'une cathode 2.1, 2.2, 2.3, d'une anode 4.1, 4.2, 4.3 et d'un électrolyte 3.1, 3.2, 3.3, intercalé entre la cathode et l'anode, et une pluralité d'interconnecteurs 5 électrique et fluidique agencés chacun entre deux cellules élémentaires adjacentes avec une de ses faces en contact électrique avec l'anode d'une des deux cellules élémentaires et l'autre de ses faces en contact électrique avec la cathode de l'autre des deux cellules élémentaires. On alimente et on distribue la vapeur d'eau à la cathode 2.1, 2.3 d'une (C1 ou C3) des deux cellules (C1, C2 ; C2, C3) élémentaires adjacentes et on alimente et on distribue du dioxyde de carbone à la cathode 2.2 de l'autre (C2) des deux cellules élémentaires (C1, C2 ; C2, C3).

Dans le réacteur de co-électrolyse selon la demande FR 12 62174, tous les compartiments cathodiques 50 dans lesquels circulent la vapeur d'eau alimentée H2O et l'hydrogène produit H$_2$ communiquent entre eux. De même, tous les compartiments cathodiques 50 dans lesquels circulent le dioxyde de carbone injecté CO$_2$ et le monoxyde de carbone produit CO communiquent entre eux, mais sont complètement isolés des compartiments 50 dédiés à la vapeur d'eau H$_2$O et à l'hydrogène produit H2. Enfin, les deux réactions d'électrolyse simultanées mais séparés produisent toutes deux de l'oxygène qui est récolté par tous les compartiments anodiques 51 qui communiquent entre eux, quelle que soit la réaction concernée.

Actuellement, lorsqu'on souhaite réaliser une méthanation, deux voies sont possibles. La première voie est celle directe, avec une unique réaction selon l'équation suivante :

$$CO_2 + 4H_2 \rightarrow CH_4 + 2H_2O.$$

La deuxième voie est celle indirecte, avec une réaction en deux étapes selon les équations suivantes :

$$CO_2 + H2 \rightarrow CO + H_2O$$

$$CO + 3H2 \rightarrow CH_4 + H_2O.$$

La méthanation est mise en oeuvre dans un réacteur au sein duquel est présent le catalyseur solide de la réaction.

L'hydrogène et le cas échéant le monoxyde de carbone peuvent être produits au préalable soit par électrolyse EHT dans un réacteur d'electrolyse 1 décrit en référence aux figures 1 à 3, soit par co-électrolyse à haute température également dans un réacteur de co-électrolyse 1 décrit ou dans un réacteur de co-électrolyse simultanée selon la demande de brevet FR 12 62174.

Ainsi, le procédé global implique l'utilisation séquentielle de deux réacteurs distincts, celui d'électrolyse/co-électrolyse et celui de méthanation, avec comme inconvénients majeurs afférents un investissement important et un coût de production élevé notamment du fait du découplage thermique entre les deux réacteurs distincts et la nécessité de comprimer en sortie du réacteur de méthanation le méthane produit afin de pouvoir le transporter soit dans les gazoducs dédiés à une pression de 80bars, soit dans les réseaux de distribution dits de moyenne pression à 4 bars.

Pour pallier ces inconvénients, les inventeurs de la présente invention ont pensé à intégrer un réacteur de méthanation avec son catalyseur solide et un électrolyseur de la vapeur d'eau à haute température (SOEC) ou un co-électrolyseur de vapeur d'eau et de dioxyde de carbone CO$_2$ au sein d'une même enceinte étanche sous pression, la pression étant celle de l'alimentation en vapeur d'eau de l'électrolyseur/co-électrolyseur, typiquement à 30 bars. Dans le cadre de l'invention, si l'on souhaite avoir du méthane en sortie qui soit à une pression supérieure, la pression d'alimentation en vapeur, et par conséquent celle au sein de l'enceinte, est à cette pression supérieure. En particulier, on peut vouloir avoir du méthane en sortie à une pression de 80 bars qui correspond à la pression rencontrée dans les gazoducs de transport de méthane : la pression d'alimentation en vapeur d'eau et dans l'enceinte est donc dans ce cas égale à 80 bars.

Ainsi, comme illustré aux figures 3 à 5, les inventeurs ont conçu un nouveau réacteur 6 pour l'obtention du méthane par catalyse hétérogène intégrant à la fois le réacteur d'électrolyse/co-électrolyse 1 à empilement de cellules d'électrolyse SOEC et le catalyseur solide nécessaire à la conversion catalytique à distance du réacteur d'électrolyse/co-électrolyse 1.

Le réacteur de méthanation 6 comporte tout d'abord un réacteur 1 d'électrolyse/co-électrolyse logé dans une enceinte 7 étanche apte à être mise sous la pression donnée à laquelle la vapeur d'eau H$_2$O d'alimentation arrive dans le réacteur 1. Tel qu'illustré aux figures 3 à 5, l'enceinte 7 du réacteur de méthanation est de forme générale cylindrique d'axe longitudinal X et le réacteur 1 est centré sur cet axe X, i.e. le centre non représenté de chacune cellules C1, C2... constituant l'empilement du réacteur 1 est sur l'axe X.

Telle qu'illustrée aux figures 3 à 5, l'enceinte étanche 7 comporte un couvercle 70, un fond 71, et une enveloppe latérale 72 assemblée à la fois au couvercle 70 et au fond 71. Le fond 71 et le couvercle 70 peuvent être assemblés sur l'enveloppe latérale 72 par un système de bride boulonnée munie d'un joint d'étanchéité.

Pour refroidir l'enceinte 7, il est prévu un circuit de refroidissement constitué d'un tuyau 73 enroulée en hélice régulière sur la paroi externe de l'enveloppe latérale 72. Ce circuit de refroidissement 73 peut avantageusement refroidir les parois internes 74 délimitant l'enceinte 7 en dessous de la température de saturation de l'eau à la pression régnant dans l'enceinte, avantageusement en dessous de 230°C à 30 bars. Ainsi, comme expliqué plus précisément ci-après, on peut avantageusement condenser la vapeur d'eau non convertie sur les parois internes 74 et ainsi récupérer indépendamment le méthane produit et la vapeur d'eau par gravité.

A l'intérieur de l'enceinte étanche 7, est agencée une cloison poreuse 8 contenant un catalyseur 80 solide de conversion de gaz de synthèse en méthane ou d'un mélange de dioxyde de carbone $CO_2$ et d'hydrogène en méthane. Le catalyseur solide peut avantageusement être, $Ni-Al_2O_3$ ou $Ni-ZrO_2$ ou celui mentionné dans la publication [2], à savoir le catalyseur bimétallique $Ni-Fe/y - Al_2O_3$ ayant des excellentes propriétés catalytiques de la méthanation sous une pression de 30 bars.

Telle qu'illustrée aux figures 3 à 5, la cloison poreuse 8 est constituée de deux parois métalliques 81, 82 formées chacune d'une tôle percée d'une pluralité de trous 83 régulièrement espacés à la fois sur la hauteur et sur la longueur de la cloison 8, la hauteur étant la dimension de la cloison selon l'axe X, la longueur étant sa circonférence autour de l'axe X. En sus de la répartition uniforme des trous 83, il est prévu qu'un trou 83 d'une des parois 81 soit en regard d'un trou 83 de l'autre des parois 82. A contrario, il peut également être prévu un décalage entre ces trous 83 d'une paroi 81 à l'autre 82.

Telle qu'illustrée également, la cloison 8 est fermée sur elle-même en formant un cylindre agencé de manière concentrique autour et à distance du réacteur 1. Enfin, un couvercle 84 distinct de celui de l'enceinte 7 ferme le volume intérieur délimité par la cloison poreuse 8. Ainsi, la présence du couvercle 84 permet de forcer le gaz à traverser le catalyseur pour sortir de l'enceinte. L'espace séparant les deux tôles 81, 82 est rempli du catalyseur 80 de conversion. Celui-ci est avantageusement sous forme de poudre qui peut être introduite dans l'espace entre les deux tôles 81, 82 avant fermeture par le couvercle 84. La fermeture du couvercle sur les tôles peut avantageusement être réalisée par soudure ou par tout autre moyen de fixation mécanique. Le(s) moyen(s) de fixation mécanique n'ont pas à être dimensionnées pour résister à un effort important car ce(s) moyen(s) n'est (ne sont) pas sollicité(s) par la pression régnant dans l'enceinte 7. Il peut s'agir par exemple d'une attache de type sauterelle, d'une vis à travers le couvercle 84 pénétrant dans la paroi 82.

Tel qu'illustré aux figures 3 à 5, un premier support 9 est agencé dans l'enceinte 7 pour supporter à la fois le réacteur d'électrolyse ou de co-électrolyse 1 et de la cloison poreuse 8 afin de les agencer à distance du fond 71 et du couvercle 70 de l'enceinte 7. Ce premier support 9 ferme en outre le volume en dessous de la cloison 8 et du réacteur 1.

Tel qu'illustré aux figures 3 à 5, il est prévu un deuxième support 10, fixé sur le premier support 9, pour supporter uniquement le réacteur d'électrolyse ou de co-électrolyse 1 afin de l'agencer en regard de la portion centrale de la cloison poreuse 8.

De préférence, le réacteur 1 est à mi-hauteur de la cloison poreuse 8, c'est-à-dire agencé en regard d'une portion situé à la moitié de la hauteur des parois 81, 82. Cela permet d'une part d'avoir un gradient thermique homogène dans le volume intérieur délimité par la paroi 81 et d'autre part d'avoir une répartition homogène des gaz ($H_2$ et CO ou $H_2$ et $CO_2$) sortant du réacteur 1 dans ce volume intérieur et donc une répartition homogène des gaz à convertir en méthane lors de leur entrée dans le catalyseur 80. Bien entendu, comme expliqué en détail ci-après, le gradient thermique entre le réacteur 1 et la cloison poreuse 8 est nécessaire du fait de la différence de température de réaction entre d'une part celle de l'électrolyse de la vapeur d'eau ou la co-électrolyse de vapeur d'eau et de CO2, avantageusement de l'ordre de 800°C, et d'autre part celle de méthanation, avantageusement autour de 400°C.

Ainsi, un agencement concentrique de la cloison poreuse 8 contenant le catalyseur de conversion 80 autour du réacteur 1, une répartition uniforme des trous 83 de passage des gaz ($H_2$ et CO ou $H_2$ et $CO_2$) sortant du réacteur 1 et un agencement du réacteur 1 à mi-hauteur de la cloison 8 contribuent à un gradient thermique très homogène dans le volume intérieur délimité par la cloison 8, son couvercle 84 et le support 9 et une répartition très homogène des gaz ($H_2$ et CO ou $H_2$ et $CO_2$) dans ce volume intérieur. Le trajet des gaz au sein du catalyseur 80 peut être relativement court, même pour une grande quantité de catalyseur présente entre les parois 81, 82, ce qui est avantageux pour la gestion thermique de la réaction de méthanation sur toute la circonférence de la cloison 8. L'épaisseur de la cloison 8, c'est-à-dire sa dimension la plus faible transversalement à l'axe X, peut donc être relativement faible devant ses autres dimensions.

Tel qu'illustré aux figures 3 à 5, la cloison 8 comporte, au sein du catalyseur solide 80, une partie de circuit de refroidissement 85 apte à refroidir la réaction catalytique de méthanation ou autrement dit à maintenir une température constante, avantageusement de 400°C pour celle-ci. En effet, la réaction de méthanation étant exothermique, le circuit de refroidissement 85 au sein du catalyseur 80 permet maintenir ce dernier à une température adaptée, de préférence proche de 400°C. Plus précisément, le circuit de refroidissement 85 peut comprendre un tuyau enroulé en hélice régulière dans l'espace entre paroi interne 81 et paroi externe 82, en étant de préférence à proximité de la paroi interne 81. Le circuit de refroidissement 85 peut contenir une huile en tant qu'agent de refroidissement et être un circuit fermé.

Tel qu'illustré aux figures 3 à 5, un tuyau d'alimentation 11 est prévu pour alimenter en vapeur d'eau sous pression et, le cas échéant en dioxyde de carbone, les cathodes du réacteur d'électrolyse ou de co-électrolyse 1. Ce tuyau 11 traverse depuis l'extérieur le fond 71 de l'enceinte 7 et le premier support 9. Il est enroulé en partie sur lui-même à proximité du réacteur 1, de préférence autour du deuxième support 10 pour surchauffer la vapeur d'eau sous pression et le cas échéant le dioxyde de carbone avant d'alimenter les cathodes, comme expliqué plus précisément ci-après.

Pour former la vapeur d'eau sous pression, il est prévu un échangeur de chaleur 12 agencé à l'extérieur de l'enceinte 7 et qui constitue un dispositif de production de vapeur d'eau ou générateur de vapeur. Pour ce faire, de l'eau liquide, comprimée au préalable à une pression donnée, dans un tuyau 13 alimente le générateur de vapeur (GV) 12. En cas de co-électrolyse par le réacteur 1, le dioxyde de carbone $CO2$ est introduit par un tuyau 14 pour être mélangé dans le GV 12 avec la vapeur d'eau formée. Il peut être envisagé d'agencer le générateur de vapeur 12 à l'intérieur de l'enceinte 7 mais pour des raisons de sécurité liées au GV (notamment la quantité de gaz en présence en cas de dépressurisation), il est préférable de l'agencer à l'extérieur comme représenté.

En tant que source de chaleur du GV 12, on peut avantageusement utiliser le circuit de refroidissement fermé 85 de la réaction de méthanation. Ainsi, comme illustré aux figures 3 à 5, le tuyau 85 en hélice régulière à l'intérieur de la cloison 8 et fermé sur lui-même traverse le fond 71 de l'enceinte et vient former le circuit primaire, i.e. celui véhiculant le fluide le plus chaud, du générateur de vapeur-échangeur 12. Autrement dit, le circuit de refroidissement 85 de la réaction de catalyse au sein de la cloison 8 constitue avantageusement le circuit de chaleur permettant de vaporiser l'eau liquide sous pression dans le GV-échangeur 12.

Tel qu'illustré aux figures 3 à 5, un tuyau 15 d'injection en dioxyde de carbone dans le volume intérieur entre le réacteur d'électrolyse 1 et la cloison poreuse 8 est prévu. Cela permet de faire une méthanation par voie directe entre l'hydrogène produit par l'électrolyse de la vapeur d'eau sous pression dans le réacteur 1 et le $CO_2$ injecté par les trous débouchant 16 du tuyau 15. Ainsi, dans cette voie directe le mélange $H_2 + CO_2$ traverse les trous 83 de la cloison 8 contenant le catalyseur 80 pour être converti en méthane. Un avantage subséquent à cette injection de $CO_2$ froid par le tuyau 15 est de permettre la gestion du gradient thermique nécessaire entre l'électrolyse dans le réacteur 1 et la catalyse au sein du catalyseur 80 dans la cloison 8.

Tel qu'illustré aux figures 3 à 5, il est prévu un tuyau 17 de récupération de méthane produit et un tuyau 18 de récupération par gravité d'eau condensée sur les parois internes 74 délimitant l'enceinte, chaque tuyau 17, 18 traversant le fond 71 de l'enceinte 7. Afin de ne pas introduire d'eau condensée dans le tuyau 17 de récupération de méthane, celui-ci fait saillie du fond 71. Au contraire, l'extrémité du tuyau 18 de récupération par gravité de l'eau condensée ne fait pas saillie du fond 71. On peut également prévoir d'agencer l'extrémité de récupération du tuyau 17 sur le couvercle 70 pour éviter à coup sûr que ledit tuyau 17 ne récupère des condensats.

On peut prévoir avantageusement de réintroduire l'eau condensée récupérée par le tuyau 18 en entrée 13 de l'eau liquide sous la même pression, du GV- échangeur de chaleur 12.

Tel que mieux illustré en figures 4 et 5, pour réaliser une diffusion homogène du dioxyde de carbone $CO_2$ injecté dans le volume intérieur délimité par la cloison poreuse 8, le tuyau 15 d'injection est enroulé sur lui-même en formant un cercle et étant percé d'une pluralité de trous 16 répartis régulièrement le long du cercle.

On peut réaliser avantageusement cette même répartition homogène dans le volume intérieur délimité par la cloison poreuse 8, pour l'hydrogène $H2$ ou le gaz de synthèse $CO + H_2$ produit dans le réacteur 1. Ainsi, tel que mieux illustré en figures 4 et 5, un tuyau 19 de récupération de l'hydrogène et le cas échéant du monoxyde de carbone produit(s) aux cathodes du réacteur 1 est prévu. Plus précisément, ce tuyau 19 de récupération est relié à la sortie des compartiments cathodiques 50 du réacteur 1 et il est enroulé sur lui-même en formant un cercle. Il est percé d'une pluralité de trous 20 répartis régulièrement le long du cercle pour diffuser de manière homogène l'hydrogène et le cas échéant du monoxyde de carbone dans le volume intérieur délimité par la cloison poreuse 8.

On indique maintenant plus précisément le fonctionnement du réacteur 6 et système de méthanation qui vient d'être décrit, en référence avec un point de fonctionnement nominal. Les conditions de fonctionnement sont les suivantes :

- injection d'eau liquide à 20°C, et comprimée à une pression de 30 bars par le tuyau 13 dans le générateur de vapeur 12;
- maintien étanche sous la pression de 30 bars de l'enceinte 7 et maintien à température constante inférieure à 230°C parois 74;
- évacuation de la vapeur d'eau, le cas échéant mélangée à du $CO_2$ injecté en 14, du GV 12 par le tuyau 11 à 300°C, à la même pression de 30 bars ;
- surchauffe de la vapeur d'eau à 300°C et 30 bars, le cas échéant mélangée à du $CO_2$ injecté en 14, dans la partie enroulée sur lui-même du tuyau 11 à proximité du réacteur 1 pour atteindre une température de 800°C en entrée de ce dernier ;
- lorsque la vapeur d'eau évacuée dans le tuyau 11 ne contient pas de $CO_2$, alors injection de $CO_2$ à température ambiante par le tuyau 15 à trous 16;

- maintien à température constante aux environs de 400°C de la cloison 8;
- passage du mélange $H_2$ + CO +$H_2O$ évacués par le tuyau 19 en sortie de réacteur de co-électrolyse 1, et/ou avec du $CO_2$ injecté par le tuyau 15, dans la cloison poreuse 8 ;
- réaction de méthanation à 400°C au sein de la cloison 8 ;
- évacuation par les trous 83 de la paroi externe 82 du méthane $CH_4$ produit et de l'eau non convertie en EHT et formée par la méthanation dans le volume délimité entre cloison 8 et enceinte 7;
- condensation de l'eau sur parois 74 délimitant enceinte 7 ;
- récupération du méthane produit sous la pression de 30 bars par le tuyau 17;
- récupération par gravité par le tuyau 18 de l'eau liquide condensée et à la pression de 30 bars ;
- réinjection de l'eau liquide récupérée à 30 bars dans le générateur de vapeur 12.

Dans des conditions de fonctionnement non nominales, il peut être prévu d'injecter du $CO_2$ à la fois par le tuyau 15 (voie directe) et par le tuyau 14 (voie indirecte).

La montée de la vapeur d'eau sous pression de 300°C à 800°C à proximité de l'électrolyseur (co-électrolyseur) 1 peut se faire uniquement par le dégagement exothermique de la réaction au sein de ce dernier. Un système de chauffe non représenté peut également servir.

Les réacteur 6 et système de méthanation qui viennent d'être décrits sont simples de réalisation avec un coût d'investissement faible. En particulier, toutes les parois 81, 82 et couvercle 84 de la cloison 8, les constituants 70, 71, 72 de l'enceinte 7, les supports 9, 10, les tuyaux 11, 13, 14, 15, 17, 18, 19, 73, 85 peuvent être réalisés à partir d'un métal relativement peu coûteux, tel que l'acier inoxydable 316L. Bien entendu, on veillera à choisir un métal adapté pour les parties devant résister aux hautes températures de l'électrolyse/co-électrolyse, typiquement à 800°C. Ainsi, pour au moins les parties de tuyau 11, 19 à l'intérieur desquels circulent des gaz à 800°C et 30 bars, on peut envisager une réalisation avec des alliages à base de nickel.

Les réacteur 6 et système de méthanation qui viennent d'être décrits permettent un coût de production plus faible que ceux de l'art antérieur, notamment du fait du couplage thermique optimisé entre les deux réactions (électrolyse/co-électrolyse et méthanation) au sein de la même enceinte 7 sous pression et du fait de l'absence d'équipement de compression de méthane, l'absence d'enceinte pression spécifique à la méthanation, l'absence de condenseur à 30bars, toutes ces fonctions étant réalisées de facto au sein de l'enceinte 7.

L'invention n'est pas limitée aux exemples qui viennent d'être décrits ; on peut notamment combiner entre elles des caractéristiques des exemples illustrés au sein de variantes non illustrées.

Ainsi, si dans l'exemple de mise en oeuvre détaillé, le réacteur 6 et système sont prévus pour réaliser la méthanation, ils peuvent tout aussi bien l'être pour obtenir du méthanol $CH_3OH$; du DME ou du diesel. Quel que soit le gaz combustible que l'on cherche à obtenir, les paramètres suivants préférés peuvent rester identiques :

- pression d'alimentation en eau liquide égale à la pression de l'enceinte 7, de l'ordre de 30 bars,
- température d'électrolyse ou de co-électrolyse de l'ordre de 800°C pour produire $H_2$+CO.

En revanche, selon le type de combustible (carburant) visé, le ratio $H_2$/CO, le choix du catalyseur 80 et la température pour la catalyse, i.e. dans la cloison poreuse 8 sont différents. Pour ce dernier paramètre, la température en cloison 8 peut être de l'ordre de 400°C pour la production du méthane $CH_4$, et de l'ordre de 250°C-300°C pour le méthanol $CH_3OH$ et le DME

## Références citées

[1]: Fabien Ocampo et al, « Methanation of carbon dioxide over nickel-based Ce0.72Zr0.28O2 mixed oxide catalysts prepared by sol-gel method», Journal of Applied Catalysis A: General 369 (2009) 90-96;
[2]: Dayan Tiang et al, « Bimetallic Ni-Fe total-methanation catalyst for the production of substitute natural gas under high pressure », Journal of Fuel 104 (2013) 224-229.

## Revendications

1. Procédé d'obtention d'un gaz combustible choisi parmi le méthane, méthanol, le diméthyléther (DME) ou le diesel par catalyse hétérogène, comportant les étapes suivantes :

    a/ une étape d'électrolyse à haute température de la vapeur d'eau $H_2O$ mise en oeuvre dans un réacteur (1) d'électrolyse logé dans une enceinte (7) étanche maintenue sous une pression donnée, étape a/ selon laquelle on alimente chaque cathode du réacteur (1) en vapeur d'eau sous la pression donnée;

b/ une étape de conversion catalytique mise en oeuvre au sein d'au moins une zone de réaction (8) agencée à distance et radialement au réacteur d'électrolyse dans la même enceinte (7) sous pression et contenant au moins un catalyseur solide (80) de la conversion, l'étape b/ étant réalisée à partir de l'hydrogène $H_2$ produit lors de l'étape a/ d'électrolyse et, de dioxyde de carbone $CO_2$ injecté dans l'espace entre le réacteur d'électrolyse et la zone de réaction radiale;

c/ une étape de récupération du gaz combustible produit et de la vapeur d'eau non convertie selon l'étape a/ et produite selon l'étape b/, dans l'espace entre ladite zone de réaction radiale et la(les) paroi(s) délimitant l'enceinte.

2. Procédé d'obtention d'un gaz combustible choisi parmi le méthane, méthanol, le diméthyléther (DME) ou le diesel par catalyse hétérogène, comportant les étapes suivantes :

a'/ une étape de co-électrolyse à haute température de la vapeur d'eau $H_2O$ et du dioxyde de carbone $CO_2$ mise en oeuvre dans un réacteur (1) de co-électrolyse logé dans une enceinte (7) étanche maintenue sous une pression donnée; étape a'/ selon laquelle on alimente chaque cathode du réacteur (1) en vapeur d'eau $H_2O$ et en dioxyde de carbone $CO_2$ sous la pression donnée;

b'/ une étape de conversion catalytique étant mise en oeuvre au sein d'au moins une zone de réaction (8) agencée à distance et radialement au réacteur de co-électrolyse dans la même enceinte (7) sous pression et contenant au moins un catalyseur solide de la conversion, l'étape b'/ étant réalisée à partir de l'hydrogène $H_2$ et du monoxyde de carbone CO produits lors de l'étape a'/ de co-électrolyse ;

c'/ une étape de récupération du gaz combustible produit et de la vapeur d'eau non convertie selon l'étape a'/ et produite selon l'étape b'/, dans l'espace entre ladite zone de réaction radiale et la(les) paroi(s) délimitant l'enceinte.

3. Procédé selon l'une des revendications précédentes, selon lequel on refroidit les parois délimitant l'enceinte à une température inférieure à la température de saturation de l'eau à la pression donnée de l'enceinte, de sorte que l'étape c/ ou c'/ consiste en une séparation du gaz combustible de l'eau condensée au sein de l'enceinte, puis une récupération du gaz combustible séparé et de l'eau condensée par gravité sur le fond de l'enceinte.

4. Procédé selon l'une des revendications précédentes constituant un procédé de méthanation.

5. Réacteur (6) pour l'obtention d'un gaz combustible choisi parmi le méthane, méthanol ou le diméthyléther (DME) par catalyse hétérogène, comportant :

- une enceinte (7) étanche apte à être mise sous pression donnée;
- un réacteur (1) soit d'électrolyse à hautes températures de la vapeur d'eau soit de co-électrolyse à hautes températures de vapeur d'eau et de dioxyde de carbone, comportant un empilement de cellules d'électrolyse élémentaires (C1, C2, C3) de type SOEC formées chacune d'une cathode (2.1, 2.2, ...), d'une anode (4.1, 4.2, ...) et d'un électrolyte (3.1, 3.2...) intercalé entre la cathode et l'anode, et une pluralité d'interconnecteurs (5) électrique et fluidique agencés chacun entre deux cellules élémentaires (C1, C2) adjacentes avec une de ses faces en contact électrique avec l'anode (4.1) d'une (C1) des deux cellules élémentaires et l'autre de ses faces en contact électrique avec la cathode (2.2) de l'autre des deux cellules (C2) élémentaires, le réacteur d'électrolyse ou de co-électrolyse étant logé dans l'enceinte et la sortie des cathodes étant débouchante à l'intérieur de l'enceinte;
- au moins une cloison poreuse (8) agencée à distance et radialement au réacteur d'électrolyse ou de co-électrolyse dans l'enceinte et contenant au moins un catalyseur solide (80) de conversion de gaz de synthèse ($H_2$+CO ou $H_2$+$CO_2$) en gaz combustible ;
- au moins un tuyau (11) d'alimentation en vapeur d'eau sous pression et, le cas échéant en dioxyde de carbone, des cathodes du réacteur d'électrolyse ou de co-électrolyse ;
- le cas échéant, au moins un tuyau (15) d'injection en dioxyde de carbone de l'espace entre le réacteur d'électrolyse et la cloison poreuse ;
- au moins un tuyau (17) de récupération de gaz combustible et/ou de vapeur d'eau,
- le cas échéant, au moins un tuyau (18) de récupération d'eau condensée sur les parois délimitant l'enceinte, chaque tuyau traversant une paroi délimitant l'enceinte.

6. Réacteur selon la revendication 5, la cloison poreuse (8) étant fermée sur elle-même en étant agencée de manière concentrique autour du réacteur d'électrolyse ou de co-électrolyse.

**7.** Réacteur selon la revendication 5 ou 6, la cloison poreuse étant constituée de deux parois (81, 82) métalliques poreuses, l'espace les séparant étant rempli au moins partiellement d'un catalyseur (80) de conversion sous forme de poudre ou de granulats.

**8.** Réacteur selon l'une des revendications 5 à 7, la cloison poreuse comportant, au sein du catalyseur solide, une partie de circuit de refroidissement (85) apte à refroidir la réaction catalytique entre l'hydrogène et le monoxyde de carbone produits en amont dans le réacteur de co-électrolyse ou entre l'hydrogène produit en amont dans le réacteur d'électrolyse et du dioxyde de carbone injecté dans l'espace entre la cloison poreuse et le réacteur d'électrolyse.

**9.** Réacteur selon l'une des revendications 5 à 8, le tuyau d'alimentation (11) étant enroulé en partie sur lui-même à proximité du réacteur d'électrolyse ou de co-électrolyse pour réchauffer la vapeur d'eau sous pression et le cas échéant le dioxyde de carbone avant d'alimenter les cathodes.

**10.** Réacteur selon l'une des revendications 5 à 9, comportant un tuyau (19) de récupération de l'hydrogène et le cas échéant du monoxyde de carbone produit(s) aux cathodes, le tuyau de récupération étant enroulé sur lui-même en formant un cercle et étant percé d'une pluralité de trous (20) répartis régulièrement le long du cercle pour diffuser de manière homogène l'hydrogène et le cas échéant du monoxyde de carbone dans l'espace entre le réacteur d'électrolyse ou de co-électrolyse et la cloison poreuse agencée de manière concentrique.

**11.** Réacteur selon l'une des revendications 5 à 10, le tuyau (15) d'injection du dioxyde de carbone étant enroulé sur lui-même en formant un cercle et étant percé d'une pluralité de trous (16) répartis régulièrement le long du cercle pour diffuser de manière homogène le dioxyde de carbone dans l'espace entre le réacteur d'électrolyse ou de co-électrolyse et la cloison poreuse agencée de manière concentrique.

**12.** Réacteur selon l'une des revendications 5 à 11, l'enceinte étanche (7) comportant une enveloppe latérale (72), un couvercle (70) et un fond (71) assemblés à l'enveloppe de manière étanche, et un premier support (9) pour supporter à la fois le réacteur (1) d'électrolyse ou de co-électrolyse et de la cloison poreuse (8) afin de les agencer à distance du fond et du couvercle de l'enceinte.

**13.** Réacteur selon la revendication 12, comportant un deuxième support (10), fixé sur le premier support (9), pour supporter uniquement le réacteur (1) d'électrolyse ou de co-électrolyse afin de l'agencer en regard de la portion centrale de la cloison poreuse, de préférence à mi-hauteur de la cloison poreuse.

**14.** Système comportant :

- un réacteur (6) selon l'une des revendications 5 à 13 ;
- un échangeur de chaleur formant un générateur de vapeur (12) pour vaporiser de l'eau liquide sous la pression donnée, l'échangeur étant agencé à l'extérieur de l'enceinte (7).


**Patentansprüche**

**1.** Verfahren zur Herstellung eines Brenngases ausgewählt aus Methan, Methanol, Dimethylether (DME) oder Diesel durch heterogene Katalyse, umfassend die folgenden Schritte:

a/ einen Schritt der Hochtemperatur-Elektrolyse des Wasserdampfes $H_2O$, der in einem Elektrolysereaktor (1) umgesetzt wird, der in einem dichten Behälter (7) untergebracht ist, der unter einem gegebenen Druck gehalten wird, Schritt a/, wobei jeder Kathode des Reaktors (1) Wasserdampf unter dem gegebenen Druck zugeführt wird,
b/ einen Schritt der katalytischen Umwandlung, der in mindestens einer Reaktionszone (8) umgesetzt wird, die im Abstand und radial zu dem Elektrolysereaktor in demselben Behälter (7) unter Druck angeordnet ist und mindestens einen festen Katalysator (80) zur Umwandlung enthält, wobei der Schritt b/ ausgehend von dem Wasserstoff $H_2$, der während des Elektrolyseschritts a/ erzeugt wird, und Kohlendioxid $CO_2$, das in den Raum zwischen dem Elektrolysereaktor und der radialen Reaktionszone eingespritzt wird, durchgeführt wird,
c/ einen Schritt des Rückgewinnens des erzeugten Brenngases und des Wasserdampfs, der nicht gemäß Schritt a/ umgewandelt und gemäß Schritt b/ erzeugt wird, in dem Raum zwischen der radialen Reaktionszone und der Wand oder den Wänden, die den Behälter begrenzt oder begrenzen.

**2.** Verfahren zur Herstellung eines Brenngases ausgewählt aus Methan, Methanol, Dimethylether (DME) oder Diesel durch heterogene Katalyse, umfassend die folgenden Schritte:

a'/ einen Schritt der Hochtemperatur-Co-Elektrolyse des Wasserdampfes $H_2O$ und des Kohlendioxids $CO_2$, der in einem Elektrolysereaktor (1) umgesetzt wird, der in einem dichten Behälter (7) untergebracht wird, der unter einem gegebenen Druck gehalten wird, Schritt a'/, wobei jeder Kathode des Reaktors (1) Wasserdampf $H_2O$ und Kohlendioxid $CO_2$ unter dem gegebenen Druck zugeführt wird,

b'1 einen Schritt der katalytischen Umwandlung, der in mindestens einer Reaktionszone (8) umgesetzt wird, die im Abstand und radial zu dem Elektrolysereaktor in demselben Behälter (7) unter Druck angeordnet wird und mindestens einen festen Katalysator der Umwandlung enthält, wobei der Schritt b'/ ausgehend von dem Wasserstoff $H_2$ und dem Kohlenmonoxid CO, die während des Schrittes der Hochtemperatur-Co-Elektrolyse a'/ erzeugt werden, durchgeführt wird,

c'/ einen Schritt des Rückgewinnens des erzeugten Brenngases und des Wasserdampfs, der nicht gemäß Schritt a'/ umgewandelt und gemäß Schritt b'/ erzeugt wird, in dem Raum zwischen der radialen Reaktionszone und der Wand oder den Wänden, die den Behälter begrenzt oder begrenzen.

**3.** Verfahren nach einem der vorhergehenden Ansprüche, wobei die Wände, die den Behälter begrenzen, auf eine Temperatur unterhalb der Sättigungstemperatur des Wassers bei dem gegebenen Druck des Behälters derart abgekühlt werden, dass der Schritt c/ oder c'/ in einem Trennen des Brenngases von dem kondensierten Wasser innerhalb des Gehäuses und dann einem Rückgewinnen des getrennten Brenngases und des kondensierten Wassers durch Schwerkraft auf dem Boden des Gehäuses besteht.

**4.** Verfahren nach einem der vorhergehenden Ansprüchen, das ein Verfahren zur Methanisierung darstellt.

**5.** Reaktor (6) zur Herstellung eines Brenngases ausgewählt aus Methan, Methanol oder Dimethylether (DME) durch heterogene Katalyse, umfassend:

- einen dichten Behälter (7), der geeignet ist, unter einen gegebenen Druck gesetzt zu werden,
- einen Reaktor (1) entweder zur Hochtemperatur-Elektrolyse des Wasserdampfes oder Hochtemperatur-Co-Elektrolyse von Wasserdampf und Kohlendioxid, umfassend einen Stapel elementarer SOEC Elektrolysezellen (C1, C2, C3), die jeweils durch eine Kathode (2.1, 2.2., ...), eine Anode (4.1, 4.2, ...) und einen Elektrolyten (3.1, 3.2, ...), der zwischen der Kathode und der Anode eingefügt ist, gebildet sind, und mehrere elektrische und fluidische Interkonnektoren (5) aufweist, die jeweils zwischen zwei benachbarten elementaren Zellen (C1, C2) angeordnet sind und mit einer ihrer Seiten in elektrischem Kontakt mit der Anode (4.1) einer (C1) der beiden elementaren Zellen stehen und mit der anderen ihrer Seiten in elektrischem Kontakt mit der Kathode (2.2) der anderen der beiden elementaren Zellen (C2) stehen, wobei der Elektrolyse- oder Co-Elektrolyse-Reaktor in dem Behälter untergebracht ist und der Ausgang der Kathoden in das Innere des Behälters mündet,
- mindestens eine poröse Trennwand (8), die im Abstand und radial zu dem Elektrolyse- oder Co-Elektrolyse-Reaktor in dem Behälter angeordnet ist und mindestens einen festen Katalysator (80) zur Umwandlung von Synthesegas ($H_2$+CO oder $H_2$+$CO_2$) in Brenngas enthält,
- mindestens ein Rohr (11) zum Zuführen von Wasserdampf unter Druck und gegebenenfalls von Kohlendioxid an die Kathoden des Elektrolyse- oder Co-Elektrolyse-Reaktors,
- gegebenenfalls mindestens ein Rohr (15) zum Einspritzen von Kohlendioxid in den Raum zwischen dem Elektrolysereaktor und der poröse Trennwand,
- mindestens ein Rohr (17) zum Rückgewinnen des Brenngases und/oder des Wasserdampfs,
- gegebenenfalls mindestens ein Rohr (18) zum Rückgewinnen des kondensierten Wassers auf den Wänden, die den Behälter begrenzen, wobei jedes Rohr eine Wand durchquert, die den Behälter begrenzt.

**6.** Reaktor nach Anspruch 5, wobei die poröse Trennwand (8) in sich selbst geschlossen ist, indem sie konzentrisch um den Elektrolyse- oder Co-Elektrolyse-Reaktor angeordnet ist.

**7.** Reaktor nach Anspruch 5 oder 6, wobei die poröse Trennwand aus zwei porösen metallischen Wänden (81, 82) gebildet ist, wobei der Raum, der sie trennt, mindestens teilweise mit einem Katalysator (80) zur Umwandlung in Form von Pulver oder von Granulaten gefüllt ist.

**8.** Reaktor nach einem der Ansprüche 5 bis 7, wobei die poröse Trennwand im Inneren des festen Katalysators einen Teil eines Kühlkreislaufs (85) aufweist, der geeignet ist, die katalytische Reaktion zwischen dem Wasserstoff und Kohlenmonoxid, die vorgelagert in dem Co-Elektrolyse-Reaktor erzeugt werden, oder zwischen dem Wasserstoff, der vorgelagert in dem Elektrolyse-Reaktor erzeugt wird, und dem Kohlendioxid, das in den Raum zwischen der porösen Trennwand und dem Elektrolysereaktor eingespritzt wird, abzukühlen.

9. Reaktor nach einem der Ansprüche 5 bis 8, wobei das Rohr zum Zuführen (11) in der Nähe des Elektrolyse- oder Co-Elektrolyse-Reaktors teilweise um sich selbst aufgerollt ist, um den Wasserdampf unter Druck und gegebenenfalls das Kohlendioxid vor dem Zuführen an die Kathoden zu erhitzen.

10. Reaktor nach einem der Ansprüche 5 bis 9, umfassend ein Rohr (19) zum Rückgewinnen des Wasserstoffs und gegebenenfalls des Kohlenmonoxids, der oder das an den Kathoden erzeugt wird, wobei das Rohr zum Rückgewinnen um sich selbst aufgerollt ist, indem es einen Kreis bildet, und mit mehreren Löchern (20) durchbohrt ist, die gleichmäßig entlang des Kreises verteilt sind, um den Wasserstoff und gegebenenfalls Kohlenmonoxid in dem Raum zwischen dem Elektrolyse- oder Co-Elektrolyse-Reaktor und der porösen Trennwand, die konzentrisch angeordnet ist, homogen zu verteilen.

11. Reaktor nach einem der Ansprüche 5 bis 10, wobei das Rohr (15) zum Einspritzen des Kohlendioxids um sich selbst aufgerollt ist, indem es einen Kreis bildet, und mit mehreren Löchern (16) durchbohrt ist, die gleichmäßig entlang des Kreises verteilt sind, um das Kohlenmonoxid in dem Raum zwischen dem Elektrolyse- oder Co-Elektrolyse-Reaktor und der porösen Trennwand, die konzentrisch angeordnet ist, homogen zu verteilen.

12. Reaktor nach einem der Ansprüche 5 bis 11, wobei der dichte Behälter (7) einen seitlichen Mantel (72), einen Deckel (70) und einen Boden (71), die an dem Mantel dicht zusammengefügt sind, und einen ersten Träger (9) aufweist, um gleichzeitig den Elektrolyse- oder Co-Elektrolyse-Reaktor (1) und die poröse Trennwand (8) zu tragen, um sie beabstandet von dem Boden und dem Deckel des Behälters anzuordnen.

13. Reaktor nach Anspruch 12, umfassend einen zweiten Träger (10), der auf dem ersten Träger (9) befestigt ist, um nur den Elektrolyse- oder Co-Elektrolyse-Reaktor (1) zu tragen, um ihn gegenüberliegend von dem Mittelabschnitt der porösen Trennwand, vorzugsweise auf halber Höhe der porösen Trennwand anzuordnen.

14. System, umfassend:

    - einen Reaktor (6) nach einem der Ansprüche 5 bis 13,
    - einen Wärmetauscher, der einen Dampfgenerator (12) bildet, um flüssiges Wasser unter dem gegebenen Druck zu verdampfen, wobei der Wärmetauscher im Inneren des Behälters (7) angeordnet ist.

**Claims**

1. A process for obtaining a combustible gas chosen from methane, methanol, dimethyl ether (DME) and diesel by heterogeneous catalysis, comprising the following steps:

    a/ a step of high-temperature electrolysis of steam $H_2O$ performed in an electrolysis reactor (1) housed in a leaktight chamber (7) maintained at a given pressure, in which step a/ each cathode of the reactor (1) is fed with steam at the given pressure;
    b/ a step of catalytic conversion performed in at least one reaction zone (8) placed at a distance from and radially to the electrolysis reactor in the same chamber (7) under pressure and containing at least one solid conversion catalyst (80), step b/ being performed using hydrogen $H_2$ produced during the electrolysis step a/ and carbon dioxide $CO_2$ injected into the space between the electrolysis reactor and the radial reaction zone;
    c/ a step of recovery of the combustible gas produced and of the steam not converted in step a/ and produced in step b/, in the space between said radial reaction zone and the wall(s) delimiting the chamber.

2. A process for obtaining a combustible gas chosen from methane, methanol, dimethyl ether (DME) and diesel by heterogeneous catalysis, comprising the following steps:

    a'/ a step of high-temperature co-electrolysis of steam $H_2O$ and carbon dioxide $CO_2$ performed in a co-electrolysis reactor (1) housed in a leaktight chamber (7) maintained at a given pressure; in which step a'/ each cathode of the reactor (1) is fed with steam $H_2O$ and carbon dioxide $CO_2$ at the given pressure;
    b'/ a step of catalytic conversion being performed in at least one reaction zone (8) placed at a distance from and radially to the co-electrolysis reactor in the same chamber (7) under pressure and containing at least one solid conversion catalyst, step b'/ being performed using hydrogen $H_2$ and carbon monoxide CO produced during the co-electrolysis step a'/;

c'/ a step of recovering the combustible gas produced and the steam not converted in step a'/ and produced in step b'/, in the space between said radial reaction zone and the wall(s) delimiting the chamber.

3. The process as claimed in either of the preceding claims, according to which the walls delimiting the chamber are cooled to a temperature below the saturation temperature of water at the given pressure of the chamber, such that step c/ or c'/ consists of a separation of the combustible gas from the water condensed in the chamber, followed by a recovery of the combustible gas separated out and of the condensed water by gravity on the bottom of the chamber.

4. The process as claimed in either of the preceding claims, constituting a methanation process.

5. A reactor (6) for obtaining a combustible gas chosen from methane, methanol and dimethyl ether (DME) by heterogeneous catalysis, comprising:

- a leaktight chamber (7) capable of being placed under a given pressure;
- a reactor (1) either for the high-temperature electrolysis of steam or for the high-temperature co-electrolysis of steam and carbon dioxide, comprising a stack of elemental electrolysis cells (C1, C2, C3) of SOEC type each formed from a cathode (2.1, 2.2, etc.), an anode (4.1, 4.2, etc.) and an electrolyte (3.1, 3.2, etc.) intercalated between the cathode and the anode, and a plurality of electrical and fluid interconnectors (5) each arranged between two adjacent elemental cells (C1, C2) with one of its faces in electrical contact with the anode (4.1) of one (C1) of the two elemental cells and the other of its faces in electrical contact with the cathode (2.2) of the other of the two (C2) elemental cells, the electrolysis or co-electrolysis reactor being housed in the chamber and the outlet of the cathodes emerging inside the chamber;
- at least one porous partition (8) placed at a distance from and radially to the electrolysis or co-electrolysis reactor in the chamber and containing at least one solid catalyst (80) for converting syngas ($H_2$+CO or $H_2$+$CO_2$) into combustible gas;
- at least one tube (11) for feeding steam under pressure and, where appropriate, carbon dioxide to the cathodes of the electrolysis or co-electrolysis reactor,
- where appropriate, at least one tube (15) for injecting carbon dioxide of the space between the electrolysis reactor and the porous partition;
- at least one tube (17) for recovering combustible gas and/or steam,
- where appropriate, at least one tube (18) for recovering water condensed on the walls delimiting the chamber, each tube passing through a wall delimiting the chamber.

6. The reactor as claimed in claim 5, the porous partition (8) being closed on itself and being placed concentrically around the electrolysis or co-electrolysis reactor.

7. The reactor as claimed in claim 5 or 6, the porous partition consisting of two porous metal walls (81, 82), the space separating them being at least partially filled with a conversion catalyst (80) in the form of powder or granulates.

8. The reactor as claimed in one of claims 5 to 7, the porous partition comprising, in the solid catalyst, part of the cooling circuit (85) suitable for cooling the catalytic reaction between the hydrogen and the carbon monoxide produced upstream in the co-electrolysis reactor or between the hydrogen produced upstream in the electrolysis reactor and carbon dioxide injected into the space between the porous partition and the electrolysis reactor.

9. The reactor as claimed in one of claims 5 to 8, the feed tube (11) being partly wound on itself close to the electrolysis or co-electrolysis reactor to heat the steam under pressure and, where appropriate, the carbon dioxide before feeding the cathodes.

10. The reactor as claimed in one of claims 5 to 9, comprising a tube (19) for recovering the hydrogen and, where appropriate, the carbon monoxide produced at the cathodes, the recovery tube being wound on itself forming a circle and being pierced with a plurality of holes (20) regularly distributed along the circle to homogeneously diffuse hydrogen and, where appropriate, carbon monoxide in the space between the electrolysis or co-electrolysis reactor and the porous partition arranged concentrically.

11. The reactor as claimed in one of claims 5 to 10, the carbon dioxide injection tube (15) being wound on itself forming a circle and being pierced with a plurality of holes (16) regularly distributed along the circle to homogeneously diffuse carbon dioxide in the space between the electrolysis or co-electrolysis reactor and the porous partition arranged concentrically.

12. The reactor as claimed in one of claims 5 to 11, the leaktight chamber (7) comprising a side envelope (72), a lid (70) and a base (71) assembled with the envelope in a leaktight manner, and a first support (9) for supporting both the electrolysis or co-electrolysis reactor (1) and the porous partition (8) so as to arrange them at a distance from the base and from the lid of the chamber.

13. The reactor as claimed in claim 12, comprising a second support (10), fixed onto the first support (9), to support only the electrolysis or co-electrolysis reactor (1) so as to arrange it facing the central portion of the porous partition, preferably half way up the porous partition.

14. A system comprising:

- a reactor (6) as claimed in one of claims 5 to 13;
- a heat exchanger forming a steam generator (12) for vaporizing liquid water at the given pressure, the exchanger being placed outside the chamber (7).

## Fig.1

$2H_2$
$(+ H_2O)$

$2O^{2-}$

$O_2$
$(+ gaz drainant)$

$2H_2O$
$(+ H_2)$

(gaz drainant)

$4e^-$       $-$  $+$       $4e^-$

1

## Fig.2

Amenée courant

$H_2 + H_2O$

5

50

$H_2O$

C2

51

5

50

$H_2O$

C1

Motif élémentaire

2.2

3.2

4.2

$O_2$

2.1

# Fig.3

# Fig.4

# Fig.5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20090289227 A
- FR 1262174
- FR 2931168

**Littérature non-brevet citée dans la description**

- **FABIEN OCAMPO et al.** Methanation of carbon dioxide over nickel-based Ce0.72Zr0.28O2 mixed oxide catalysts prepared by sol-gel method. *Journal of Applied Catalysis A: General,* 2009, vol. 369, 90-96
- **DAYAN TIANG et al.** Bimetallic Ni-Fe total-methanation catalyst for the production of substitute natural gas under high pressure. *Journal of Fuel,* 2013, vol. 104, 224-229